# EUROPEAN PATENT APPLICATION

(11) **EP 4 723 123 A1**
(43) Date of publication of application: **08.04.2026**
(21) Application number: 25206229.4
(22) Date of filing: 02.10.2025
(51) Int. Cl.: G16H 20/10, G16H 50/20

(54) **OPEN LOOP INTELLIGENT DOSING**

(30) Priority: 07.10.2024 US 202463704328 P; 16.09.2025 US 202519330568
(71) Applicant: Medtronic MiniMed, Inc., Northridge, CA 91325-1219 (US)
(72) Inventor: STONE, Michael P., Northridge, CA 91325 (US); MIKHNO, Arthur, Northridge, CA 91325 (US); TURKSOY, Kamuran, Northridge, CA 91325 (US); GROSMAN, Benyamin, Northridge, CA 91325 (US)
(74) Representative: Zimmermann & Partner Patentanwälte mbB

(57) **Abstract**

Systems, devices, and techniques are disclosed for providing personalized insulin dosage recommendations for a patient. In some aspects, a method includes generating a physiological model for estimating glucose values of the patient based on historical data, the historical data comprising glucose value history and insulin dosage history for a time window during which the patient consumed a plurality of meals. The method also may include estimating, using the physiological model and the historical data, an impact of the plurality of meals on the glucose values of the patient, and receiving a first meal size indication of a first meal for consumption by the patient. The method may also include outputting a first meal bolus insulin dose recommendation based on the impact of the plurality of meals on the glucose values of the patient and the first meal size indication.

## Description

### RELATED APPLICATIONS

This application claims the benefit of the filing date of provisional U.S. Patent Application No. 63/704,328, filed October 7, 2024, entitled "OPEN LOOP INTELLIGENT DOSING," which is assigned to the assignee hereof, and incorporated herein in its entirety by reference.

### TECHNICAL FIELD

The present disclosure relates generally to medicine administering and tracking systems, devices, and processes.

### BACKGROUND

Diabetes mellitus, also referred to as diabetes, is a condition characterized by elevated blood glucose levels resulting from insufficient insulin production or impaired insulin action. Management of the condition often requires administration of insulin to regulate glucose levels and support metabolic balance. Insulin may be delivered using various approaches, including syringes, prefilled insulin pens, and continuous subcutaneous infusion pumps. Patients frequently administer insulin themselves, adjusting dosage based on factors such as blood glucose measurements, diet, and physical activity. Advances in glucose monitoring, including continuous glucose monitoring systems, have enabled closer integration of real-time glucose data with insulin administration practices.

### SUMMARY

Techniques for determining insulin dosage recommendations are provided. The techniques may be practiced with a processor-implemented method, a system comprising one or more processors and one or more processor-readable media, and/or one or more non-transitory processor-readable media.

An example method of providing personalized insulin dosage recommendations for a patient, according to this disclosure, comprises generating a physiological model for estimating glucose values of the patient based on historical data, the historical data comprising glucose value history and insulin dosage history for a time window during which the patient consumed a plurality of meals. The method further comprises estimating, using the physiological model and the historical data, an impact of the plurality of meals on the glucose values of the patient, and receiving a first meal size indication of a first meal for consumption by the patient. The method further comprises outputting a first meal bolus insulin dose recommendation based on the impact of the plurality of meals on the glucose values of the patient and the first meal size indication.

An example system, according to this disclosure, comprises one or more processors and one or more processor-readable media storing instructions which, when executed by the one or more processors, cause performance of generating a physiological model for estimating glucose values of the patient based on historical data, the historical data comprising glucose value history and insulin dosage history for a time window during which the patient consumed a plurality of meals. The one or more processor-readable media further store instructions which, when executed by the one or more processors, cause performance of estimating, using the physiological model and the historical data, an impact of the plurality of meals on the glucose values of the patient, and receiving a first meal size indication of a first meal for consumption by the patient. The one or more processor-readable media further store instructions which, when executed by the one or more processors, cause performance of outputting a first meal bolus insulin dose recommendation based on the impact of the plurality of meals on the glucose values of the patient and the first meal size indication.

An example of one or more non-transitory processor readable media, according to this disclosure, stores instructions which, when executed by one or more processors, cause performance of: generating a physiological model for estimating glucose values of a patient based on historical data, the historical data comprising glucose value history and insulin dosage history for a time window during which the patient consumed a plurality of meals. The one or more non-transitory processor readable media also store instructions which, when executed by one or more processors, cause performance of estimating, using the physiological model and the historical data, an impact of the plurality of meals on the glucose values of the patient, and receiving a first meal size indication of a first meal for consumption by the patient. The one or more non-transitory processor readable media also store instructions which, when executed by one or more processors, cause performance of outputting a first meal bolus insulin dose recommendation based on the impact of the plurality of meals on the glucose values of the patient and the first meal size indication.

Further disclosed herein are systems, devices, and techniques for providing personalized insulin dosage recommendations for a patient. In some aspects, a method includes generating a physiological model for estimating glucose values of the patient based on historical data, the historical data comprising glucose value history and insulin dosage history for a time window during which the patient consumed a plurality of meals. The method also may include estimating, using the physiological model and the historical data, an impact of the plurality of meals on the glucose values of the patient, and receiving a first meal size indication of a first meal for consumption by the patient. The method may also include outputting a first meal bolus insulin dose recommendation based on the impact of the plurality of meals on the glucose values of the patient and the first meal size indication.

### BRIEF DESCRIPTION OF THE DRAWINGS

The above and other aspects and features of the disclosure will become more apparent in view of the following detailed description when taken in conjunction with the accompanying drawings, wherein like reference numerals identify like elements.
FIG. 1 is a diagram of an example of a therapy system, in accordance with aspects of the present disclosure.
FIG. 2 is a diagram of an example of a drug delivery device, in accordance with aspects of the present disclosure.
FIG. 3 is a block diagram of an example of a computer system, which can be utilized in embodiments as described herein.
FIG. 4 is a block diagram of a procedural flow or providing open-loop intelligent dosing, in accordance with aspects of the present disclosure.
FIG. 5 is a flow diagram of an example process of performing retrospective meal tagging, in accordance with aspects of the present disclosure.
FIG. 6 is a flow diagram illustrating an example of historical meal impact learning, in accordance with aspects of the present disclosure.
FIG. 7 is a graph of example meal impact percentile curves, which can be utilized in embodiments as described herein.
FIG. 8 is an illustration of aspects of the procedural flow of FIG. 4 related to basal recommendation, in accordance with aspects of the present disclosure.
FIG. 9 is an illustration of aspects of the procedural flow of FIG. 4 related to correction bolus recommendation, in accordance with aspects of the present disclosure.
FIG. 10 is a message flow diagram illustrating messages communicated between devices, in accordance with aspects of the present disclosure.
FIG. 11 is a block diagram of a modified procedural flow showing a variation to the procedural flow of FIG. 4 that may be implemented, in accordance with aspects of the present disclosure.
FIG. 12 is a flow diagram of a method of providing personalized insulin dosage recommendations for a patient, in accordance with aspects of the present disclosure.

### DETAILED DESCRIPTION

Therapeutic substances (e.g., insulin) may be delivered to a diabetic patient to manage, for example, type I or type II diabetes. Delivering appropriate amounts of a therapeutic substance at appropriate times can help maintain glucose levels within a target range (e.g., a euglycemic range) in the body to prevent hyperglycemia or hypoglycemia conditions. As used herein, the term "bolus," "bolus dose," "meal dose," meal bolus," or "meal bolus insulin dose" refers to a discrete dose of insulin delivered over a relatively short period of time, typically for the purpose of counteracting a rise in blood glucose associated with food intake or for correcting an elevated blood glucose level. A bolus may be manually initiated by a user or automatically administered by an insulin delivery system in response to sensed or predicted glucose values. The term "correction bolus" or "correction dose" refers to a bolus administered specifically to reduce blood glucose levels that are above a target range, which may be independent of meal intake. A correction bolus may be calculated based on a user's insulin sensitivity factor, the difference between measured glucose and target glucose, or other dosing algorithms implemented by an insulin delivery system. The term "basal" or "basal dose" refers to the delivery of insulin at a substantially continuous or periodic rate over an extended time interval, generally for the purpose of maintaining blood glucose levels within a desired range in the absence of meal-related glucose excursions. Basal delivery may be provided by long-acting insulin formulations administered through injection or by programmable insulin infusion from an insulin pump or other delivery device (which may administer fast-acting insulin). Therapy involving manually injecting insulin (e.g., long-acting basal and short-acting bolus) multiple times a day may be referred to as multiple daily injections (MDI) therapy.

Together, bolus (including correction bolus) and basal insulin delivery may be employed in various regimens to maintain glycemic control in individuals with diabetes. Accurate insulin dosage is critical for maintaining blood glucose within a desired range and preventing complications associated with both hyperglycemia and hypoglycemia. Determining an appropriate dose generally involves consideration of multiple factors, including current blood glucose level, anticipated carbohydrate intake, insulin sensitivity, fasting glucose, and activity level. Because these variables may change throughout the day and differ across individuals, the process of calculating insulin requirements can be complex and highly individualized. Insulin delivery described herein can generally be referred to as employing a "treat to range" (T2R) strategy in which a patient's glucose levels are treated to fall within a generally acceptable or "normal" range. That said, as noted elsewhere, alternative embodiments may be directed toward other types of drug delivery. As such, alternative embodiments may employ a "treat to target" (T2T) strategy in which a specific, measurable treatment goal for a disease is targeted and treatment is adjusted to achieve and maintain it within a tolerance threshold (e.g., 1%, 3%, 5%, etc., which may depend on the type of disease, personalized treatment plan, and/or other factors). Moreover, in some embodiments for insulin delivery, a T2T strategy may be employed in certain circumstances, depending on desired functionality.

Current insulin dosage calculators are typically designed to provide estimates based on standardized parameters such as insulin-to-carbohydrate ratios and correction factors. In open-loop systems, where a user (typically the recipient of the insulin, referred to herein as a "patient") manually administers insulin in response to the calculator's recommendation, these tools may not account for dynamic physiological variations between users or even within the same user over time. For example, changes in stress, illness, hormonal cycles, or circadian rhythms can significantly affect insulin sensitivity, yet such influences are often not incorporated into conventional dosage algorithms, so dosages are usually not titrated as a patient's insulin needs change over time. As a result, while these calculators provide helpful guidance, they may not fully reflect individualized metabolic variations that impact optimal insulin dosing.

Embodiments described herein address these and other issues by providing intelligent dosing recommendations that are personalized to a specific patient. More specifically, embodiments provide techniques for determining patient-specific insulin recommendations based on modeling the impact of specific meals based on historical data of the patient. According to some embodiments, glucose value history and insulin dosage history can be used to determine when a person has consumed meals, and model the impact of each of those meals on the glucose values of the patient. In some implementations, the various impacts of each of these meals may be analyzed to determine parameters that enable patients to easily categorize future meals (as "small," "medium," or "large," for example) and/or allow meals to be more accurately modeled based on an estimate of carbohydrates (carb count). Embodiments can be used to recommend a basal dose, meal bolus, and/or correction bolus, which may be based on meal impact modeling. Moreover, some embodiments implement outcome-based adjustments that monitor outcomes of doses (e.g., in addition to meal modeling) for increased accuracy. It is noted that, even though the techniques herein envision providing a dose or other recommendation, alternative embodiments are envisioned in which the recommended dose is delivered automatically (e.g., when the techniques are implemented within a pump or other automated insulin delivery system).

The embodiments described herein provide one or more of the following advantages, which may be seen as improvements to the field of drug administration, particularly insulin delivery. By processing the historical data of the patient to determine a meal impact response for the patient, embodiments provide for personalized, highly accurate dosage recommendations based on meal categorization that reflects actual meals consumed by a patient. Further, by enabling the importation of this historical data from other sources (e.g., closed-loop devices, such as insulin pumps), embodiments enable the personalization of dosage recommendations without an additional need for a learning period. Further, by adapting the model-based dosage recommendations to accommodate a user input comprising carb count, embodiments can enable a "backwards compatibility" of these personalized models to traditional user input. These and other advantages will become apparent to a person of ordinary skill in the art in view of the embodiments described below, which are provided after an overview of systems/components that may be used to implement such embodiments.

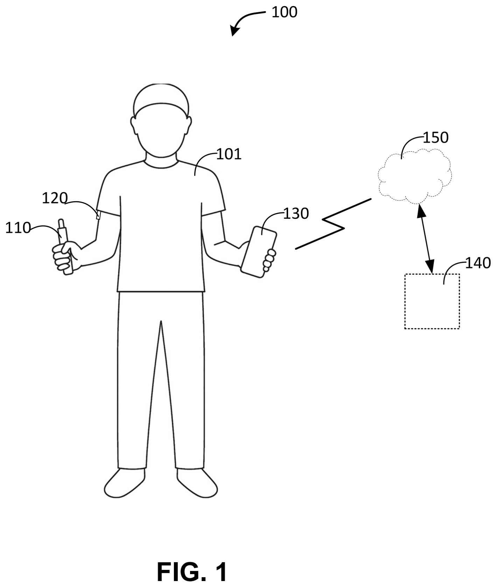

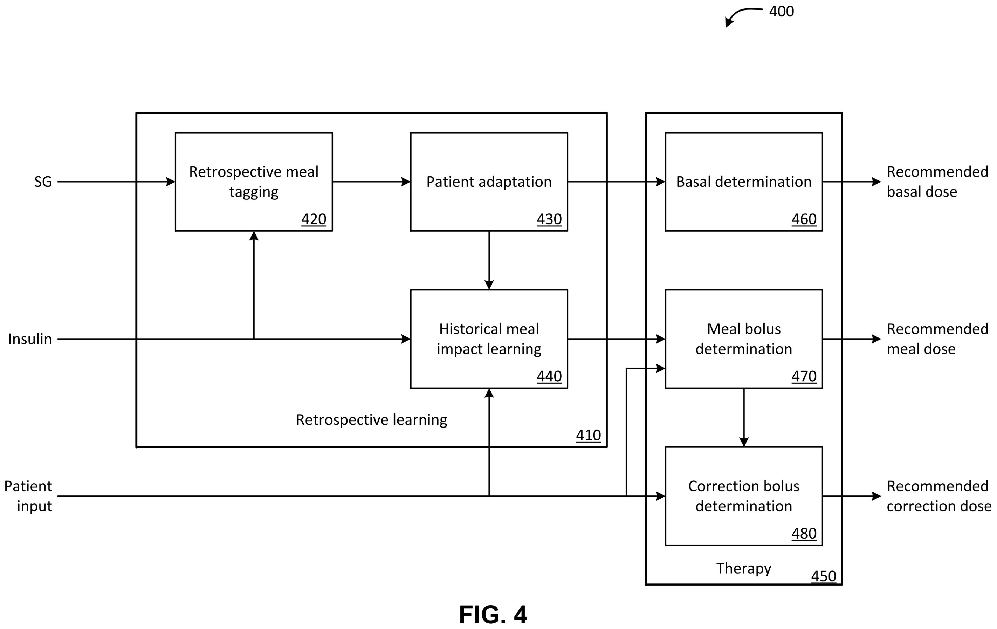

**FIG.** 1 depicts an example therapy system 100, according to an embodiment. Components of the therapy system 100 may be used by a user, or patient 101, and may implement one or more of the functions described herein, including functions illustrated in FIGS. 4-12. The system 100 may be an insulin therapy system. The depicted therapy delivery 100 may be considered an "open loop" system because patient drug dosage is determined based on various relevant factors, and then the insulin dosage is manually administered to the patient 101. (E.g., in contrast to "closed loop" systems with automated drug administration systems.) The depicted therapy system 100 includes a drug delivery device 110, a sensor device 120, and a computing device 130. According to some embodiments, the system 100 may further include optional components (as depicted by dashed lines), including a remote or cloud computing system 140, accessible via one or more networks 150. As with other figures herein, FIG. 1 is provided as a non-limiting example, and alternative embodiments may have an alternative arrangement of components, combined or separate components, or include other such variations apparent to a person of ordinary skill in the art. Note that the locations of each component shown in FIG. 1 are merely one example. For example, sensor device 120 is depicted on the upper arm of the patient 101; however, in some implementations, sensor device 120 may be on the abdomen, upper buttocks, thigh, lower back, flank area, or elsewhere.

The drug delivery device 110 may comprise a pen injection apparatus or a similar medicine delivery device, in communication with the computing device 130. The drug delivery device 110 is operable to select, set, and/or dispense a dose of the medicine for dispensing. In some implementations, the computing device 130 comprises a smartphone, tablet, and/or wearable computing device, such as a smartwatch, smart glasses, etc. In some implementations, the computing device 130 may be in communication with other local computing devices (e.g., personal computers, laptops, tablets, etc., not shown) and/or the remote computing system 140. The computing device 130 executes an application (or "app") associated with the drug delivery device 110 of the therapy system 100, which can monitor and/or control functionalities of the drug delivery device 110 and to provide a dose calculator and/or decision support modules that can calculate and recommend a dose of the medicine for the patient 101 to administer using the drug delivery device 110. The recommended dose may be further informed by input from the sensor device 120.

In some embodiments, the sensor device 120 is used to monitor one or more health metrics of the patient user. Examples of health metric data monitored by the sensor device 120 include analytes and/or biomarkers, such as glucose, heart rate, blood pressure, user movement, or others. In some implementations, the sensor device 120 is a wearable sensor device, such as a continuous glucose monitor (CGM), that obtains transcutaneous or blood glucose measurements processed to produce continuous glucose values. For example, the continuous glucose monitor can include a glucose processing module implemented on a stand-alone display device and/or implemented on the computing device 130, which processes, stores, and displays the continuous glucose values for the patient 101. The output of the sensor device 120 may comprise "sensed data" as defined below.

Depending on desired functionality, the sensor device 120 may use one or more different types of sensors (not shown). Sensors can include, for example, electrochemical sensors, electrical sensors, and/or optical sensors. As persons skilled in the art will understand, an electrochemical sensor may be configured to respond to the interaction or binding of a biological marker to a substrate by generating an electrical signal based on a potential, conductance, and/or impedance of the substrate. The substrate may include a material selected to interact with a particular biomarker, such as glucose. The potential, conductance, and/or impedance may be proportional to a concentration of the particular biomarker. In the case of electrical sensors, and as persons skilled in the art will understand, an electrical sensor may be configured to respond to an electrical biosignal by generating an electrical signal based on an amplitude, frequency, and/or phase of the electrical biosignal. The electrical biosignal may include a change in electric current produced by the sum of an electrical potential difference across a tissue, such as the nervous system, of the person 101. In some embodiments, the electrical biosignal may include portions of a potential change produced by the heart of the patient 101 over time, e.g., recorded as an electrocardiogram, that are indicative of a glucose level of the patient 101. In the case of optical sensors, as persons skilled in the art will understand, an optical sensor may be configured to respond to the interaction or binding of a biological marker to a substrate by generating an electrical signal based on a change in luminance of the substrate. For example, the substrate may include a material selected to fluoresce in response to contact with a selected biomarker, such as glucose. The fluorescence may be proportional to the concentration of the selected biomarker.

As used herein, the term "sensed data" shall mean and include the information represented by a sensor signal or by a pre-processed sensor signal (e.g., from the sensor device 120). In some embodiments, sensed data may include glucose levels in a patient 101, acceleration of a part of the patient 101, heart rate of the patient 101, temperature of the patient 101, and/or geolocation (e.g., GPS location) of the patient 101, among other things. The sensor device 120 may communicate sensed data to the drug delivery device 110 and/or computing device 130 (e.g., via one or more wireless communication links). The use of sensed data will be described later herein. References to sensor glucose (SG) in the embodiments described within this disclosure may comprise sensed data.

Components of the system 100 may communicate with each other in one or more of a variety of ways. Communication links may comprise, for example, a wired connection and/or a wireless connection. In the case where two devices are located in the same device housing, the communication link may include, for example, wires, cables, and/or communication buses on a printed circuit board, among other things. In the case where two devices are separated from each other in different device housings, the communication links may be wired and/or wireless connections. Wired connections may include, without limitation, an Ethernet connection, a USB connection, and/or another type of physical connection. Wireless connections may include, without limitation, a cellular connection, a Wi-Fi connection, a Bluetooth^{®} connection, a mesh network connection, and/or another type of connection using a wireless communication protocol. Some embodiments of the communication links may use direct connections, such as Bluetooth^{®} connections, and/or may use connections that route through one or more networks or network devices (not shown), such as an Ethernet network, a Wi-Fi network, a cellular network, a satellite network, an intranet, an extranet, the Internet, and/or the Internet backbone, among other types of networks. Various combinations of wired and/or wireless connections may be used for the system 100.

The computing device 130 provides processing capabilities and may be implemented in various ways. As noted, in some embodiments, the computing device 130 may be a consumer device such as a smartphone, wearable device, etc., or may be a special-purpose device (e.g., a portable control device) provided by, for example, the manufacturer of the drug delivery device 110. In some embodiments, the computing device 130 may be "processing circuitry" (defined below) that is integrated with another device, such as the drug delivery device 110. In some embodiments, the computing device 130 may be secured to the patient 101 (e.g., to the body or clothing of person 101), may be at least partially implanted into the body of patient 101, and/or may be held by the patient 101. In some embodiments, computing device 130 may be configured to execute one or more operations of processes described herein with respect to FIGS. 4-12. The computing device 130 may be referred to herein as a "user device," a term that is used synonymously with consumer device. Additionally, although the terms "user" and "patient" may be referred to herein synonymously, in some instances, a user of the "user device" (computing device 130) may be different from the "patient" to whom a drug is administered. Thus, references such as "user input" or "patient input" may refer to input by either a user or patient, which may or may not be the same person, depending on the application.

According to some embodiments, the computing device 130 can be used to obtain, process, and/or display data that can be used to relate to the patient's health condition, including a condition for which a drug delivered by the drug delivery device 110 is used to treat. In an illustrative example, the computing device 130 is operable to track the patient's location; the patient's physical activity, including step count, movement distance, and/or intensity, estimated calories burned, and/or activity duration; and/or the patient's interaction pattern with the computing device 130. An app executed by the computing device 130 can aggregate and process the contextual data to generate decision support outputs to guide and aid the patient 101 in using the drug delivery device 110 and/or managing their behavior to promote better health outcomes in treating his/her health condition.

For each of the embodiments of the computing device 130, the computing device 130 may include various types of logic circuitry, including, but not limited to, microprocessors, microcontrollers, digital signal processors (DSPs), application specific integrated circuits (ASICs), field-programmable gate arrays (FPGAs), central processing units (CPU), graphics processing units (GPU), programmable logic devices, memory (e.g., random access memory, volatile memory, non-volatile memory, etc.), or other discrete or integrated logic circuitry, as well as combinations of such components. The term "processing circuitry" may generally refer to any of the foregoing logic circuitry, alone or in combination with other logic circuitry, or any other circuitry for performing computations.

Aspects of the drug delivery device 110, the sensing device 120, and the computing device 130 have been described above. One or more of these devices may include a user interface (not shown) that presents information to the patient 101 and/or receives information from the person 101. The user interface may include a graphical user interface (GUI), a display device, a keyboard, a touchscreen, a speaker, a microphone, a vibration motor, buttons, switches, and/or other types of user interfaces. Persons skilled in the art will recognize various types of user interfaces that may be used, and all such user interfaces are contemplated to be within the scope of the present disclosure. For example, where the computing device 130 is a consumer device such as a smartphone, tablet computer, laptop computer, or the like, the user interfaces would include a display device, a physical and/or virtual keyboard, and/or audio speakers provided by such consumer devices, among other things. In some embodiments, a user interface may notify the patient 101 of sensed data (e.g., glucose level) and/or insulin delivery data (e.g., rates of historic, current, or future insulin delivery) and may present alerts to the patient 101. In some embodiments, a user interface may receive inputs from the patient 101, which may include, for example, information regarding drug administration (e.g., insulin injection time and dosage) to the patient 101, and/or a meal indication, among other things. The descriptions and embodiments above regarding user interfaces are merely provided as examples, and other types and other uses of user interfaces are contemplated to be within the scope of the present disclosure.

Aspects of the therapy system 100 are described below. Further aspects and details may be described in United States Patent Pub. Nos.: US 2022/0395644 A1 and US 2024/0331830 A1; and United States Patent Nos.: 11,369,743; 11,563,485; and 12,128,219; the entire contents of each of the foregoing United States Patent Publications and Patents are hereby incorporated by reference herein.

With continuing reference to FIG. 1, the remote or cloud computing system 140 may be a proprietary remote/cloud computing system or a commercial cloud computing system including one or more server computing devices. The remote/cloud computing system 140 may provide additional computing resources on demand as needed when the computing resources of a client computing device (e.g., the computing device 130) are not sufficient. The computing device 130 and the remote/cloud computing system 140 may communicate with each other through a communication link that may traverse one or more communication networks 150. The communication networks 150 may include, without limitation, an Ethernet network, Wi-Fi network, a cellular network, a satellite network, an intranet, an extranet, the Internet, and/or the Internet backbone, among other types of networks. Persons skilled in the art will recognize implementations for the remote/cloud computing system 140 and how to interface with such systems through various types of networks. For example, the remote/cloud computing system 140 may include an array of processing circuitry (defined above) and may execute machine-readable instructions. Such implementations, interfaces, and networks are contemplated to be within the scope of the present disclosure.

An example therapy system has been described above. For convenience, the description below may primarily refer to an insulin delivery system as an example of the therapy delivery system. However, it is intended that any aspect, embodiment, or description relating to an insulin delivery system shall be applicable to a therapy delivery system that delivers a therapy other than insulin.

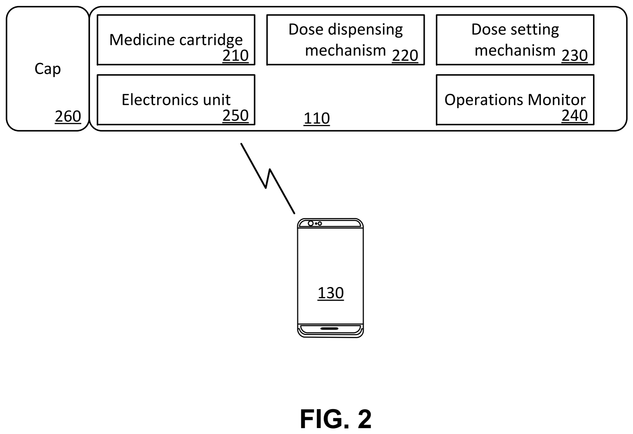

**FIG. 2** is a diagram of an example embodiment of the drug delivery device 110 of the therapy system 100. According to some embodiments, the drug delivery device 110 may comprise a pen injection apparatus, such as an insulin pen. The drug delivery device 110 is structured to have a body that contains the medicine cartridge 210 (e.g., which can be replaceable). The drug delivery device 110 is structured to include a dose dispensing mechanism 220 to dispense (e.g., deliver) the medicine contained in the medicine cartridge 210 out of the drug delivery device 110; a dose setting mechanism 230 to select and/or set the dose to be dispensed; an operations monitoring mechanism 240 to determine that the drug delivery device 110 is being operated and/or to monitor the operation of the dose being dispensed (e.g., such as a switch and/or sensor, or an encoder); and an electronics unit 250 that can include a processor, a memory, a battery or other power source, and a transmitter, a receiver, and/or transceiver. As illustrated, the drug delivery device 110 may be configured to communicate with the computing device 130 wirelessly via the electronics unit 250, according to some embodiments. As also illustrated, the drug delivery device 110 may include a cap 260 to provide a physical mechanism for protecting one or more components of the drug delivery device 110 and/or the patient when the drug delivery device 110 is not in use.

In some implementations of the system 100, for example, to use the drug delivery device 110, the patient first dials up a dose using a dose knob. The dose knob of the drug delivery device 110 can be included as part of the dose setting mechanism and/or the dose dispensing mechanism. For example, the dose may be adjusted up or down prior to administration of the dose. When the patient applies a force against a dose dispensing button (e.g., presses against the dose dispensing button that is caused to protrude outward from the device's body upon dialing the dose using the dose knob), a pushing component (e.g., also referred to as a 'plunger') of the dose dispensing mechanism is depressed against an abutment of the medicine cartridge loaded in the drug delivery device 110 to cause the drug delivery device 110 to begin to dispense the medicine, in which the quantity dispensed is in accordance with that set by the dose setting mechanism. In such implementations, the operations monitoring mechanism of the drug delivery device 110 will begin to sense movement of a rotating component or shaft that drives the plunger, for example, in which the movement is sensed through an encoder. In some examples, the encoder can be configured to sense the rotation of a component that is coupled to the drive shaft, and as the drive shaft rotates, the plunger moves linearly; therefore, by sensing the rotation of the component, the movement of the drive shaft and the plunger is sensed. Movement of the encoder may be detected as data processed by a processor of the electronics unit of the drug delivery device 110, which can be used to measure the dose. In some implementations, the processor can then store the size of the dose along with a time stamp for that dose. In some implementations, the drug delivery device 110 can then transmit the dose and related information to the computing device 130. In such implementations, when the dose is transmitted, the data associated with the particular transmitted dose is marked in the memory of the drug delivery device 110 as transmitted. In such implementations, if the dose was not yet transmitted to the computing device 130, then the data associated with the dose will be transmitted at the next time a successful communication link between the drug delivery device 110 and the computing device 130 is established.

The operations monitoring mechanism of the drug delivery device 110 can include a sensor that can utilize any method of sensing rotary or linear movement. Non-limiting examples of such sensors include rotary and linear encoders, Hall effect and other magnetic based sensors, linearly variable displacement transducers, or any other appropriate method of sensing known in the art.

The dose dispensing mechanism of the drug delivery device 110 can include a manually powered mechanism or a motorized mechanism. In either case, a force (e.g., either produced by the patient or by an electrically-powered motor) pushes on the plunger of the dose dispensing mechanism to, in turn, force a receiving plunger of the medicament vial or cartridge to deliver the specific amount of the medicament. In some implementations, for example, the dose dispensing mechanism can be adjusted to deliver the dose over a different period of time. In one example, the dose dispensing mechanism can be operated such that the plunger is pushed in by an adjustable tension spring or change the speed of the motor to inject the dose over a time frame (e.g., 1 s, 5 s or other) to aid in reducing the pain of dosing. In one example, the dose dispensing mechanism can be operated over a much longer period of time, e.g., to better match the dynamics of carbohydrates, which can be like an extended bolus with a pump.

An app executed by the computing device 130 associated with the drug delivery device 110 provides a user interface to allow the patient to manage his/her health-related data. In some implementations, for example, the computing device 130 can be configured to control some functionalities of the drug delivery device 110. In some implementations, for example, the computing device 130 includes the patient's existing smartphone, tablet, or wearable computing device. In some implementations, for example, the computing device 130 is an independent portable device that the patient may carry on his/her person. In one example embodiment of an independent portable computing device 130, the computing device 130 includes a data processing unit, a wireless communication unit to allow the device to communicate with the drug delivery device 110, and a display unit.

Although a drug delivery device 110 may comprise an insulin pen or other types of insulin injection systems as indicated herein, embodiments are not so limited. Techniques disclosed herein may apply to a wide variety of drug delivery devices used for the delivery of insulin or other drugs. This can include, for example, simple syringes, injection pens or other manual insulin delivery systems, pumps (e.g., insulin pumps, of which may include insulin pump(s) that utilize an automated insulin delivery system), inhalers, jet injectors, implantable devices, and the like. As a person of ordinary skill in the art will appreciate, the types of device used may reflect the type of drug administered.

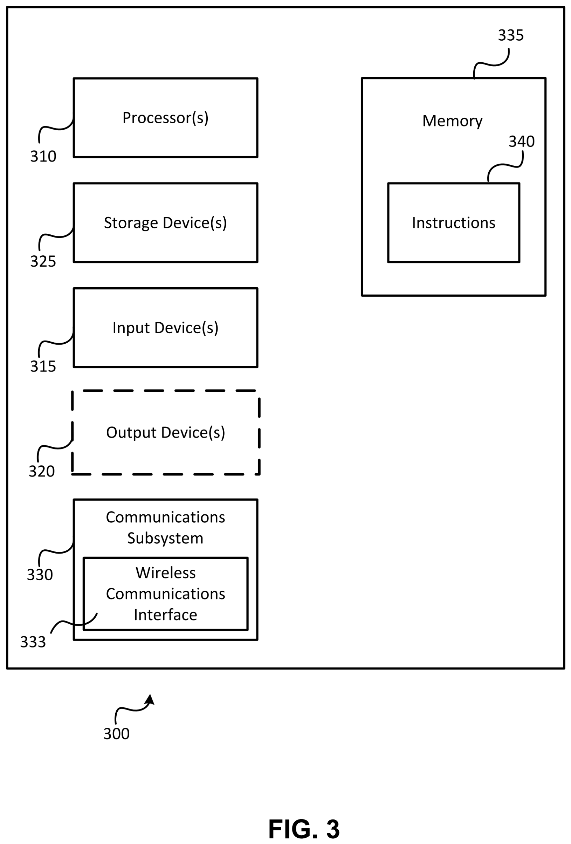

**FIG.** 3 is a block diagram of an embodiment of a computer system 300, which can be utilized in embodiments as described herein to implement one or more types of electronic devices described herein. It should be noted that FIG. 3 is meant only to provide a generalized illustration of various components, any or all of which may be utilized as appropriate. In addition, it can be noted that components illustrated by FIG. 3 can be localized to a single device (e.g., computing device 130, remote/cloud computing system140) and/or distributed among various networked devices, which may be disposed at different geographical locations.

In some embodiments, the computer system 300 may include hardware elements that can be electrically coupled via a bus (or may otherwise be in communication, as appropriate). The hardware elements may include processor(s) 310, which may comprise, without limitation, one or more microcontroller(s), one or more microprocessor(s), one or more general-purpose processors, one or more special-purpose processors (such as digital signal processing chips, graphics acceleration processors, and/or the like), and/or other processing structure, which can be configured to perform one or more of the methods or functionalities described herein.

In some embodiments, the computer system 300 also may include one or more input devices 315, which may comprise, without limitation, button elements, a microphone, a glucose sensor, and/or the like. The computer system 300 may also include one or more output devices 320, which may comprise, without limitation, a display device, a speaker, a buzzer, and/or the like.

In some embodiments, the computer system 300 may further include one or more non-transitory storage devices 325, which can include, without limitation, local and/or network accessible storage, and/or may comprise, without limitation, a disk drive, a drive array, an optical storage device, a solid-state storage device, such as a read-access memory (RAM) and/or read-only memory (ROM), which can be programmable, flash-updateable, and/or the like. Such storage devices may be configured to implement any appropriate data stores, including, without limitation, various file systems, database structures, and/or the like. Such data stores may include database(s) and/or other data structures used to store and administer messages and/or other information to be sent to one or more other components or external devices.

In some embodiments, the computer system 300 may also include a communications subsystem 330, which may implement wireless communication technologies managed and controlled by a wireless communication interface 333. Additionally or alternatively, communications subsystem 330 may implement wired technologies (such as Ethernet, coaxial communications, universal serial bus (USB), or the like). The wireless communication interface 333 may comprise one or more wireless transceivers that may send and receive wireless signals (e.g., signals according to Bluetooth^{®}, Bluetooth Low Energy (BLE)). Thus, the communications subsystem 330 may comprise a modem, a network card (wireless or wired), an infrared communication device, a wireless communication device, and/or a chipset, and/or the like, which may enable the computer system 300 to communicate with any device discussed with respect to FIG. 1, including drug delivery device 110, sensor device 120, computing device 130, and/or a remote computing system 140 as described herein. Hence, the communications subsystem 330 may be used to receive and send data (e.g., sensor glucose (SG) and/or other sensed data, insulin delivery information) as described in the embodiments herein.

In some embodiments, the computer system 300 will further comprise a working memory 335, which may comprise a RAM or ROM device, as described above. Software elements, shown as being located within the working memory 335, may comprise computer-readable and computer-executable instructions 340; device drivers; executable libraries; and/or other code, which may comprise computer programs used in various embodiments and/or may be designed to implement methods and/or configure systems in accordance with embodiments described herein. Merely by way of example, one or more operations described with respect to the methods or functionalities discussed above might be implemented as code and/or instructions executable by a computer (and/or a processor within a computer). Such code and/or instructions can be used to configure and/or adapt a general-purpose computer (or other device) to perform one or more operations in accordance with the described methods.

In some embodiments, a set of these instructions and/or code may be stored on a non-transitory computer-readable storage medium, such as the storage device(s) 325 described above. In some cases, the storage medium might be incorporated within a computer system, such as computer system 300. In other embodiments, the storage medium might be separate from a computer system (e.g., a removable medium, such as an optical disc) and/or provided in a downloadable installation package, such that the storage medium can be used to program, configure, and/or adapt a general-purpose computer with the instructions and/or code stored thereon. These instructions might take the form of executable code, which is executable by the computer system 300, and/or might take the form of source and/or installable code, which, upon compilation and/or installation on the computer system 300 (e.g., using any of a variety of generally available compilers, installation programs, compression/decompression utilities, etc.), then takes the form of executable code.

As noted, the system 100 and components illustrated in FIGS. 1-3 can be used to implement an open-loop system for administering insulin or other drugs to a patient. Embodiments herein can enhance the system 100 by implementing techniques by which those recommendations are personalized, based on historical data of the patient. As such, embodiments can take into account the patient's unique physiology, rather than provide dosage calculations based on generic algorithms. FIG. 4, discussed below, provides an overview of these techniques.

**FIG. 4** is a block diagram of a procedural flow 400 for providing open-loop intelligent dosing, according to some embodiments. Although this and other embodiments are specific to insulin dosage, they are not necessarily so limited. That is, although embodiments herein specify the use of SG, insulin, and meals, alternative embodiments may utilize additional or alternative biomarkers, drugs, and events (e.g., announced by a user/patient or otherwise detected) to implement techniques described herein for dosage delivery of other types of drugs. Alternative embodiments can include, for example, embodiments directed toward blood pressure regulation (e.g., monitoring blood pressure and providing antihypertensives in view of events such as high salt intake, stress, or missed medication), Parkinson's disease (e.g., monitoring tremor amplitude or neural activity and providing levodopa or dopamine agonists in view of events such as medication wearing off or stress), respiratory conditions such as asthma or Chronic Obstructive Pulmonary Disease (COPD) (e.g., monitoring oxygen saturation, airway resistance, or exhaled nitric oxide and providing bronchodilators or corticosteroids in view of events such as allergen exposure, exertion, or infection), cardiac arrhythmia management (e.g., monitoring electrocardiogram (ECG) irregularities and providing antiarrhythmic drugs in view of conduction disturbances), autoimmune or inflammatory disorders (e.g., monitoring cytokine levels and providing biologics or corticosteroids in view of flare-up events such as infection or environmental triggers), chemotherapy-associated nausea (e.g., monitoring biomarkers or gastric motility and providing antiemetic drugs in view of chemotherapy infusion events), and the like.

Further, the functionality of the various components illustrated in FIG. 4 may be executed on one or more computing devices, which may be local to the user, in the cloud, or a combination thereof. Referring to FIG. 1, for example, the procedural flow 400 may be performed by an app executed by the computing device 130 of FIG. 1, the remote computing system 140, or a combination thereof. It is also noted that, although sensor glucose (SG) is used in the embodiment illustrated in FIG. 4 and other embodiments in this disclosure, the embodiments are not so limited. Other types of glucose values (e.g., blood glucose (BG), interstitial glucose (IG) plasma glucose, capillary glucose, white blood glucose) or metrics derived therefrom (e.g., glucose management indicator (GMI), time and range (TIR), time above/below range (TAR/TBR), mean amplitude of glycemic excursions (MAGE)) may be used additionally or alternatively, in some embodiments.

The procedural flow 400 may begin with retrospective learning 410, which enables the procedural flow 400 to provide personalized dosage recommendations by leveraging historical data as training data. According to some embodiments, retrospective learning 410 can leverage data imported from one or more other devices. For example, historical data may be imported from an insulin pump that stores historical SG values (e.g., from a CGM) and insulin dosage history spanning a period of time (referred to herein as a "look back period" or simply a "window of time"). Additionally, or alternatively, insulin dosage history may be imported from a pen injection apparatus or other drug delivery device 110 or insulin administration apparatus. According to some embodiments, a patient may be able to manually input at least some of this historical data (e.g., insulin dosage history) via, for example, a user interface of an app executed by, and/or a web portal accessible via, the patient's mobile phone, tablet, computer, etc.

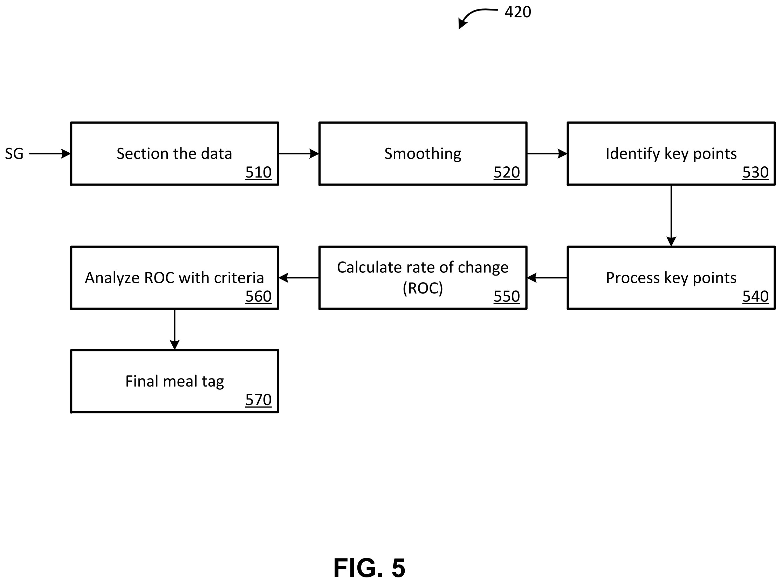

The retrospective meal tagging 420 is a process by which the historical data is analyzed to determine meal times (or, more specifically, times at which the patient first began consuming a meal) within the historical data, then updating or supplementing the historical data to include these meal times. The identification of meal times can be based, for example, on SG local minimum and local maximum points in historical SG values, where a meal time is identified in correlation with a rise in local SG values. Different meal tagging algorithms may be used, depending on desired functionality. An example of a meal detection algorithm that may be used, according to some embodiments, may be found in United States Patent no. 11,806,137, which is incorporated by reference herein. Additionally, FIG. 5, discussed below, provides an overview of one such example.

With meals identified in the historical data, the process may then perform patient adaptation 430. Patient adaptation 430 comprises data processing of the historical data to identify personalized physiological parameters of the patient, such as insulin sensitivity, fasting glucose, fasting insulin levels, and the like. According to some examples, these determinations can be done using clinical methods to analyze blood glucose in view of insulin dosage history. For example, insulin sensitivity may be based on analysis of total daily dose over time, fasting glucose may be based on SG or BG, and/or fasting insulin may be established with a physiological model of insulin action. According to some embodiments, fasting insulin can include a combination of fast-acting and long-acting insulin physiological models to establish the insulin in the body when the patient is determined to be fasting. In some embodiments, fasting period determination may be made based on a combination of distance from prior announced or detected meals, variability in glucose, variability in insulin based on the physiological models, and/or an acceptable glucose range.

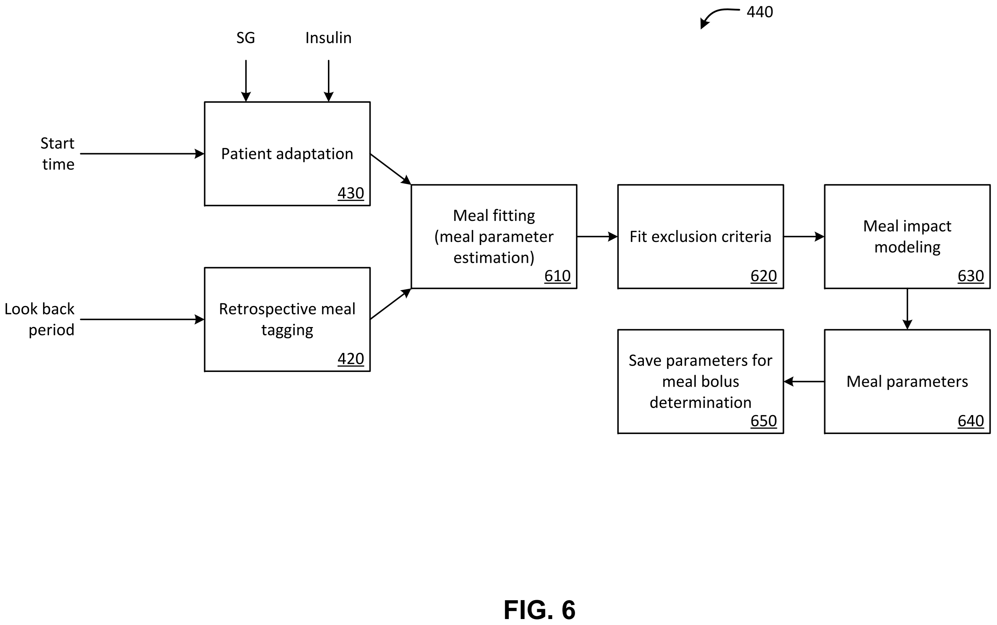

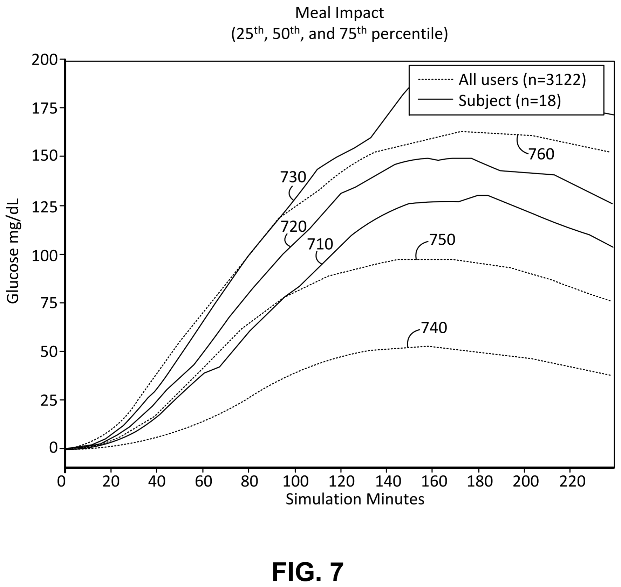

The final aspect of retrospective learning 410 comprises historical meal impact learning 440. Building on knowledge of personalized physiological parameters gained during patient adaptation 430, historical meal impact learning models the impact on the glucose for each meal identified in the historical data. Particularly, according to some examples, historical meal impact learning 440 comprises generating curves for each meal (e.g., based on a physiological model based on a set of mathematical equations), indicating the impact each meal has on glucose levels over time, while subtracting (e.g., based on determined insulin sensitivityand/or fasting glucose and/or fasting insulin) the insulin impact bolus (correction or meal) or basal insulin in the system based on a physiological model of total insulin taken or delivered recently or over many days. (Many days may be considered because a long-acting insulin dose impact may take several days to stabilize.) Additional details regarding meal impact learning 440 are provided below, with respect to FIGS. 6 and 7.

With patient adaptation 430 and historical meal impact learning 440 operations performed, the retrospective learning 410 aspect of the procedural flow 400 is complete, and the procedural flow 400 may then provide recommendations based on the results of the retrospective learning 410 as part of the therapy aspect 450 of the procedural flow 400. However, it can be noted that although the operations in retrospective learning 410 may initially be based on imported data from different sources (e.g., as part of a migration from a closed-loop system to an open-loop system), the operations in retrospective learning 410 may be ongoing, according to some embodiments. For example, retrospective learning 410 may be based on historical data obtained from an insulin pump or priori history of a patient using a digital pen injection device, based on a time window extending backwards over a period of time (e.g., 7 days, 14 days, 21 days, 28 days, etc.), or a number of meals (e.g., 15 meals, 20 meals, 25 meals, etc.) to provide one or more initial recommended insulin doses. As time passes, the window of time advances and new meals are added to the historical data (and, according to some embodiments, old data is purged), enabling the retrospective learning 410, and ultimately recommended insulin doses, to take into account any new developments or changes in the patient's physiology or meal consumption behavior. As such, the operations in retrospective learning 410 may be performed (e.g., separately or together) periodically (e.g., every number of days, hours, weeks, etc.) or based on a schedule (e.g., a certain time of day, day of the week, etc.), for example. This may depend on relevant factors such as the rate at which new information is obtained, processing resources of the device executing the retrospective learning 410, and the like.

Returning to the therapy aspect 450 of the procedural flow 400, operations may be performed for a basal determination 460, a meal bolus determination 470, and a correction bolus determination 480. As described in more detail below, basal determination 460 may comprise an operation to determine a daily dose or multiple daily doses (e.g., of a long-acting insulin) to get the patient to a fasting target glucose. Basal determination may also be used to set a pump basal rate, pump basal schedule, and/or pump basal target(s), where the insulin used by the pump is a fast-acting insulin. This process automatically detects fasting glucose from the data, which is used to provide long-acting insulin recommendations. Meal bolus determination 470 may comprise a recommended meal dose based on a meal size (e.g., small, medium, large), meal type (e.g., breakfast, lunch, dinner, snack), or announced carb amount announced by the patient, and the personalized meal impact and physiological model projection based on the historical meal impact learning 440. According to some embodiments, the recommended meal dose may also determine the nadir tolerance, which allows bolus projection to drop below a target if within an allowable value below a baseline projection, in which the learned meal impact is incorporated as additional information into the projection. Additional details on how projections may be constructed and used, according to some embodiments, may be found Grosman, B., Roy, A., Lintereur, L., Turksoy, K., Benedetti, A., Cordero, T. L., ... & Cohen, O. (2024), "A peek under the hood: explaining the MiniMed™ 780G algorithm with meal detection technology," Diabetes Technology & Therapeutics, 26(S3), 17-23, referred to herein as "the Grosman '24 paper," which is incorporated by reference herein. Correction bolus determination 480 may comprise an additional insulin dose recommendation provided after a meal, again based on a physiological model projection from historical meal impact learning 440. According to some embodiments, meal model states and/or nadir tolerance may be used in the correction bolus determination 480. Again, the determination of these therapy recommendations is described in more detail below.

**FIG. 5** is a flow diagram of an example process for performing retrospective meal tagging 420, according to one or more embodiments. Here, as noted, glucose values (e.g., SG) for the historical window of time are analyzed to determine meal times, each corresponding to a time at which the patient began consuming a meal. It can be noted that, in some examples and implementations, historical data may include meal times that have been manually labeled by a patient. However, the retrospective meal tagging process 420 of FIG. 5 can work with or without such manually tagged meals, automating the process of identifying and tagging meals.

The process 420 can begin with the functionality illustrated at blocks 510 and 520, in which the preprocessing operations of sectioning and smoothing the data are performed. These particular steps involve partitioning the data into continuous sections and performing filtering (e.g., averaging) in preparation for subsequent data processing, but additional or alternative preprocessing steps may be performed in alternative embodiments. At block 530, local minimum and maximum glucose values are identified in the preprocessed data, and these points are processed at block 540 to identify single meals, removing points in proximity and pairing local minimum with local maximum values. Operations 550 and 560 involve calculating and analyzing a rate of change (e.g., increase in glucose value over time), respectively, to determine whether a meal was consumed. According to some embodiments, this can involve determining whether the rate of change (ROC) exceeds a threshold value (e.g., a threshold increase in mg/ml over time) indicative of a meal and whether the time difference exceeds a threshold amount of minutes indicative of a meal. According to some examples, an ROC threshold value for meal determination may be 0.2 mg/dL or 0.24 mg/dL per minute for a predetermined period of time (e.g., 25 minutes, 30 minutes, 35 minutes, etc.). In some examples, an ROC threshold value for meal determination may fall within a broader range of between be 0.1-3 mg/dL per minute. Other embodiments may have ROC thresholds that differ from these examples. Finally, if the data satisfy the criteria for a meal, the data are tagged as such, as indicated at block 570. As an example, if the historical data indicates a local minimum SG value of 95 mg/dL at 1 pm, followed by a local maximum of 150 mg/dL at 2 pm, where a ROC between the local minimum and maximum values is 0.9167 mg/dL per minute (exceeding a threshold value of 0.5 mg/dL per min), then the time of the local minimum value, 1 pm, can be identified as a meal time for that particular meal.

**FIG. 6** is a flow diagram illustrating an example of historical meal impact learning 440, according to one or more embodiments. As illustrated and noted above, historical impact learning can utilize the outputs of patient adaptation 430 (e.g., insulin sensitivity, fasting glucose, fasting insulin) and retrospective meal tagging 420 (e.g., the indication of meal start times within the historical data) to model the impact of the identified meals for the patient. These inputs can be used to perform meal fitting, as indicated at block 610.

Meal fitting 610 is a process of meal parameter estimation for use by a physiological model. As noted elsewhere, the physiological model used for historical meal impact learning may comprise a set of differential equations or analytical equations that model insulin and meal impact on glucose, given a set of personalized parameters and meal and insulin logs. These equations may be determined, for example, based on concepts disclosed in Grosman, B., Wu, D., Miller, D., Lintereur, L., Roy, A., Parikh, N., & Kaufman, F. R. (2018), "Sensor-augmented pump-based customized mathematical model for type 1 diabetes," Diabetes technology & therapeutics, 20(3), 207-221; Grosman, B., Wu, D., Parikh, N., Roy, A., Voskanyan, G., Kurtz, N., ... & Vigersky, R. (2021), "Fast-acting insulin aspart (Fiasp®) improves glycemic outcomes when used with MiniMedTM 670G hybrid closed-loop system in simulated trials compared to NovoLog®," Computer Methods and Programs in Biomedicine, 205, 106087; and the Grosman '24 paper. These references are herein referred to collectively as "the Grosman papers," and are incorporated by reference herein. The Grosman papers provide examples of physiological modeling for diabetes, including meal models, that may be used for historical meal impact determination. (Alternative embodiments may use additional or alternative types of models, including alternative mathematical models, machine learning models, and the like.) Using glucose values (e.g., SG) during a meal, including the rate of change, the measured dynamics of conversion or absorption of carbohydrates from a consumed meal into the body of the patient are used to optimize a model such that the projected glucose of the model is tuned to fit the data. Put differently, meal parameters used by the physiological model are adjusted until the glucose value projections made by the model for meals consumed within the historical data fit those of the data. Additionally, in some embodiments, meal fitting may be performed sequentially, such that the impact of one meal on the next meal is considered.

The fit exclusion criteria operation at block 620 is a process by which this fit is analyzed to determine whether these projections are within a tolerable threshold and/or whether there are other reasons to exclude a particular meal projection from meal impact modeling. According to some embodiments, traditional techniques for measuring a model's predictions against data can be used to perform fitting analysis, such as mean absolute relative difference (MARD), mean squared error (MSE), or coefficient of determination (R²), or any combination of these, and meals can be excluded for which projections fail to fit the data within a threshold (e.g., less than 15% under MARD and/or less than 0.5 under R²). According to some embodiments, quality metrics additionally may be used to evaluate the goodness of fit, for example, Akaike information criterion (AIC) and the like. Additional exclusion criteria may be implemented, such as if there is no recorded bolus within a threshold amount of time (e.g., 1.5, 2, 2.5 hours, etc.), or if a meal occurs before the look-back period (e.g., more than 30, 45, or 60 days prior).

The meal impact modeling 630 uses the meal fitting to then determine the impact of each meal (not excluded at block 620) on the glucose values of the patient. In particular, the physiological model for the patient is used to determine a meal impact for each meal as a profile of glucose values over time, without the insulin impact of a corresponding bolus. These glucose value profiles may be referred to herein as "meal impact curves" or "meal impact distributions." According to some embodiments, point-wise calculations for 25^{th}, 50^{th}, and 75^{th} percentile curves can then be generated from the meal impact curves. Examples of these percentile curves are illustrated in FIG. 7.

**FIG. 7** is a graph on which 25^{th}, 50^{th} and 75^{th} percentile meal impact curves are plotted. The plots illustrated in FIGS. 7 are provided as an example, and represent curves for both a large number of people and a specific individual, helping illustrate how personalized curves can deviate from larger averages. In particular, plots 710, 720, and 730 are plots corresponding to a particular individual, respectively representing the 25^{th}, 50^{th}, and 75^{th} percentile curves of the 18 meals analyzed for that individual. As noted below, these 25^{th}, 50^{th}, and 75^{th} percentile curves can be used as a basis for setting meal parameters corresponding to meal size indications (e.g., respective "small," "medium," and "large" meals). As used herein, the term "meal size indication" refers to an indication of a meal size that can include traditional size designations (e.g., "small," "medium," and "large") meal type (e.g., snack, breakfast, lunch, dinner, etc.), carb count, and the like. These meal size indications may be received via user/patient input (e.g., a meal "announcement" via an app executed by a computing device 130) or automatically determined via an algorithm or machine-learning model, which may use information such as meal descriptions (e.g., text or audio descriptions of food content or ingredients), sensor data, images (e.g., photo(s) of a meal, plate, contents, or ingredients), and/or other types of information to generate a meal size indication.

For illustration purposes, the individualized plots 710, 720, and 730 can be compared with plots 740, 750, and 760, representing 25^{th}, 50^{th}, and 75^{th} percentile curves for a larger population of users. As can be seen, the meal impact curves deviate from those of the larger population in a significant manner, suggesting personalized insulin dosage recommendations could be particularly useful for the individual. It is noted, however, that the plots 740, 750, and 760 are provided here for comparison, and not necessarily used in the meal impact learning or modeling used in the embodiments discussed above.

Returning to FIG. 6, the functionality at block 640 comprises determining meal parameters based on the results of the meal impact modeling of block 630. As previously noted, according to some embodiments, 25^{th}, 50^{th}, and 75^{th} percentile curves can be used for the basis of setting meal parameters for respective small, medium, and large (SML) indications of meal size. For example, the model parameters that cause the personalized physiological model to most closely fit the 25^{th} percentile curve can be selected as the meal parameters to use for glucose projections and insulin dosage recommendations when a user inputs the consumption of a "small" meal. These model parameters may vary, depending on the type of physiological model used. However, according to some embodiments, these model parameters may include insulin sensitivity , meal impact gain, meal impact time constant, or the like. Equations describing a meal compartments model may be found in the Grosman papers previously cited, where meal parameters may comprise parameters of the meal compartments model. Similarly, parameters that cause the personalized physiological model to most closely fit the 50^{th} and 75^{th} percentile curves can be selected as the meal parameters to use for glucose projections and insulin dosage recommendations when a user inputs meal size indications indicative of the consumption of a "medium" or "large" meal, respectively. Of course, alternative embodiments may allow for "extra small" or "extra large" meals (e.g., based on different percentile curves, such as 10^{th} or 15^{th} percentile for "extra small" meals or 85^{th} or 90^{th} percentile for "extra large" meals), snacks, specific meals (e.g., breakfast, lunch, dinner), other meal types when determining a set of preselected meal size indications for the user to use when entering meal consumption for the determination of a bolus dosage, and different meal types may be associated with different curves, which may further be based on user input (e.g., a user may indicate that a "snack" may most closely correspond with a "medium" meal rather than a "small" or "extra small" meal). This type of personalization can help a patient customize a set of predetermined meal size indications based on meals actually consumed by the patient, facilitating meal entry input by the patient (e.g., via an app) in an intelligent manner.

According to some embodiments, the meal impact modeling at block 630 can also allow a traditional carb count input. For example, an initial set of carb count values may be associated with corresponding percentile curves (e.g., 25^{th}, 50^{th}, and 75^{th} percentile curves), and corresponding parameters may be determined to best fit those percentile curves. For example, if a user determines that typical carb counts for small, medium, and large meals are 15 g, 45 g, and 75 g, respectively, these carb counts can be associated with 25^{th}, 50^{th}, and 75^{th} percentile curves, respectively. Parameters for carb count values that differ from the initial set can be extrapolated therefrom. Using the previous example, if a user inputs 63 g as the carb count for an announced meal (where 63 g falls between the 45 g of the 50^{th} percentile curve for medium meals and the 75 g of the 75^{th} percentile curve for large meals), embodiments may approximate a meal curve for a meal having a carb count of 63 g based on either or both of the 50^{th} and 75^{th} percentile curves. The approximation may be based on a difference between the announced carb count (e.g., 63 g) and carb counts for the one or more curves used for the approximation (e.g., 45 g and/or 75 g). As another example, a ratio between the median meal curve (e.g., the 50^{th} percentile curve) and the average carb count entry may be determined, then used to scale up or down meal parameter values based on different carb count input. For example, if the user inputs a carb count of 54 g, which is 20% more than the 45 g average carb count for a meal, the meal parameter values may be scaled by 20%. It can be noted that embodiments may use any percentile and any statistic (e.g., median or average) for the ratio, depending on desired functionality. For example, a median meal curve may be used for a median carb count input, a 25th percentile meal curve may be used for a 25th percentile carb count input, and so forth. According to some embodiments, if historical data includes carb count information, then percentile curves may be mapped to specific carb counts based on historical carb count data.

At block 650, the meal parameters determined at block 640 are then saved. These parameters can then be used based on subsequent meal announcements (e.g., inputting a meal size indication) by the patient to determine meal bolus. For example, for embodiments in which SML meal parameters are determined based on patient meal consumption and saved, they can be used when a patient announces a meal (e.g., via an app executed by the patient's mobile phone) to determine a meal bolus to recommend to the patient. In another embodiment, the patient's entered meal sizes or carb amounts may be used to determine the meal curve corresponding to that meal size or carb amount. For example, all meals labeled as "small" by the user may be analyzed separately (from "medium" and "large" meals) to determine percentiles within "small" meals. Similarly, for carb amounts, meal curves within, e.g., 20-30g, may be analyzed separately from meal curves for different carb amounts. This process can be repeated for various categories (breakfast, lunch, dinner, extra small, small, large, 21-30g, 31-40g, etc.) until all meal data is covered. Additionally, or alternatively, time of the day may be considered when modeling meals, such that meals occurring within a typical night (12-6 am), breakfast (e.g., 6-11 am), lunch (11 am -4 pm), and dinner (4 pm-12 am) are analyzed separately. This may provide a more accurate model based on food content (macronutrients), carb sensitivity changes over the day, and types of meals consumed at different time periods.

According to some embodiments, meal parameters may be used to track the meal-on-board over time (e.g., glucose absorbed from the meal over time). The remaining meal-on-board may be included in the calculation of future meal, correction, and remedial boluses (e.g., as described above with respect to blocks 470 and 480 of FIG. 4). For example, meal parameters from the historical model used to calculate a meal bolus at 12 pm may be used 1 hour later to take the remaining meal-on-board into account for estimating additional insulin need (e.g., as part of correction bolus determination 480). Thus, according to some embodiments, meal-on-board persists from meal to meal, such that the previous meal in the system is taken into account for calculating the dose of the next meal.

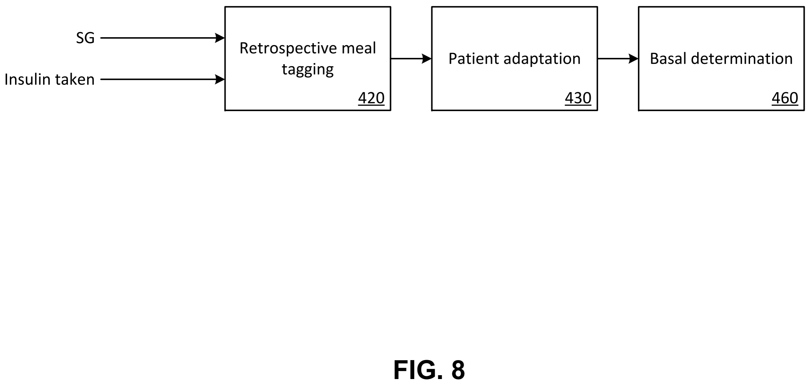

**FIG. 8** is an illustration of aspects of the procedural flow 400 of FIG. 4 related to basal determination 460. Here, retrospective meal tagging 420 and patient adaptation 430 may be performed as described above to identify meal times within the historical data. These meal times can be used filter out non-fastening periods when the algorithm estimates the patient's typical fasting glucose and fasting insulin levels. These points can be used to determine physiological parameters of the patient, such as fasting glucose and fasting insulin. Historical data can also be used to determine the insulin sensitivity of the patient. These physiological parameters can then be used to calculate a basal dose (e.g., a daily dose of long-acting insulin) that the patient should take to reach a target glucose value. This calculation can be performed using a physiological model of the patient, traditional basal calculation formulas, etc. In some embodiments, bolus and basal insulin history may be processed through physiological insulin models to establish plasma insulin levels available in the body at the time of fasting. This fasting insulin, alone or in combination with fasting glucose level, may be used to determine the daily basal insulin need required to achieve a fasting target glucose. According to some embodiments, a fasting glucose value may be determined for a patient periodically (e.g., on a daily basis), and an updated, personalized recommended basal dose can be provided accordingly.

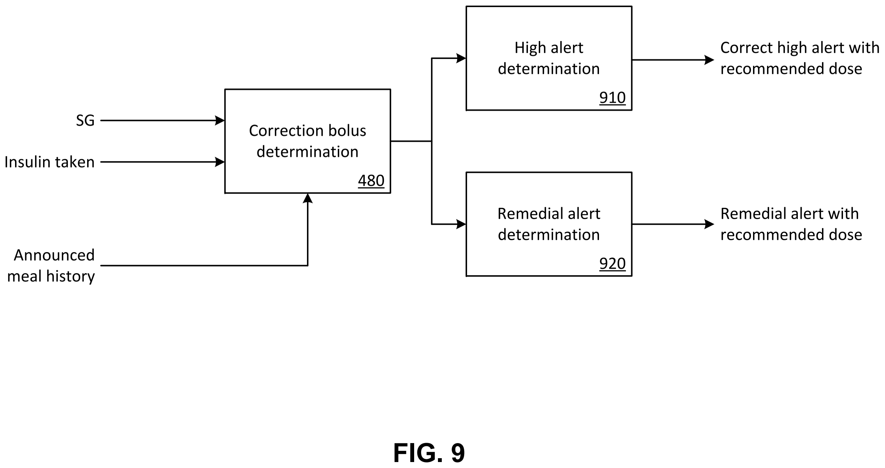

**FIG. 9** is an illustration of the correction bolus determination operation 480 of the procedural flow 400 of FIG. 4. As a person of ordinary skill in the art will appreciate, a correction bolus may comprise a bolus taken subsequent to a meal bolus, if further correction is needed after the meal bolus is taken. Thus, the determination of whether a correction bolus is needed may occur at some threshold time after a meal bolus is taken (e.g., 45 minutes, 60 minutes, 75 minutes, etc., or as specified in a user setting). The correction bolus determination operation 480 may comprise calculating a bolus periodically (e.g., every minute, 2 minutes, 5 minutes, 10 minutes, 15 minutes, etc.), which may correspond with a rate at which glucose value information (e.g., SG) from a sensor (e.g., CGM) is updated. According to some embodiments, the correction bolus determination operation 480 may calculate this bolus based on previous meal states from a historical meal model of the patient. For example, the patient announced the consumption of a "small" meal approximately an hour prior, and took the recommended dose that was based on the historical meal model, the active meal decay may be tracked and used in the periodic calculation of bolus previously described. It may output a high alert or a remedial alert if the calculated bolus exceeds a threshold (e.g., 0.5 U).

The high alert determination 910 may comprise a high-priority alert that occurs when high alert thresholds are met. Specifically, if the recommended bolus is at or above a threshold (e.g., 0.25 U, 0.5 U, or 0.75 U, or another user-selected threshold) and glucose values are at or above a threshold (e.g., SG >= 175 mg/dl, 180 mg/dl, or 185 mg/dl, or another user-selected threshold), the high alert determination 910 may then output a correct high alert with a recommended dose. According to some embodiments, the high alert determination 910 may be performed only after a threshold period of time has passed since the previous bolus (e.g., a meal bolus) was taken. Again, depending on desired functionality, this period of time may be predetermined (e.g., 30 minutes, 45 minutes, 1 hour, 2 hours, etc.) or may be determined by a user setting.

The remedial alert determination 920 may comprise a lower-priority alert that occurs when remedial thresholds are met. Specifically, if the recommended bolus is at or above a threshold (e.g., 0.25 U, 0.5 U, or 0.75 U, or another user-selected threshold), but glucose values are below a high-alert threshold (e.g., SG < 175 mg/dl, 180 mg/dl, or 185 mg/dl, or another user-selected threshold), the remedial determination 920 may then output a correct remedial alert with a recommended dose. According to some embodiments, similar to the high alert determination 910, the remedial alert determination 920 may be performed only after a threshold period of time has passed since the previous bolus (e.g., a meal bolus) was taken (e.g., 30 minutes, 45 minutes, 1 hour, 2 hours, etc.) or may be determined by a user setting.

According to some embodiments, the functionality of blocks 910 and 920 may be combined to provide a single dose recommendation (not shown in FIG. 9). For example, a single "dose now" alert may be generated, if the recommended bolus is at or above a threshold (e.g., 0.25 U, 0.5 U, or 0.75 U, or another user-selected threshold). This alert can monitor for insulin in all SG ranges, and activate based on whether insulin need is above the threshold (e.g., regardless of SG levels).

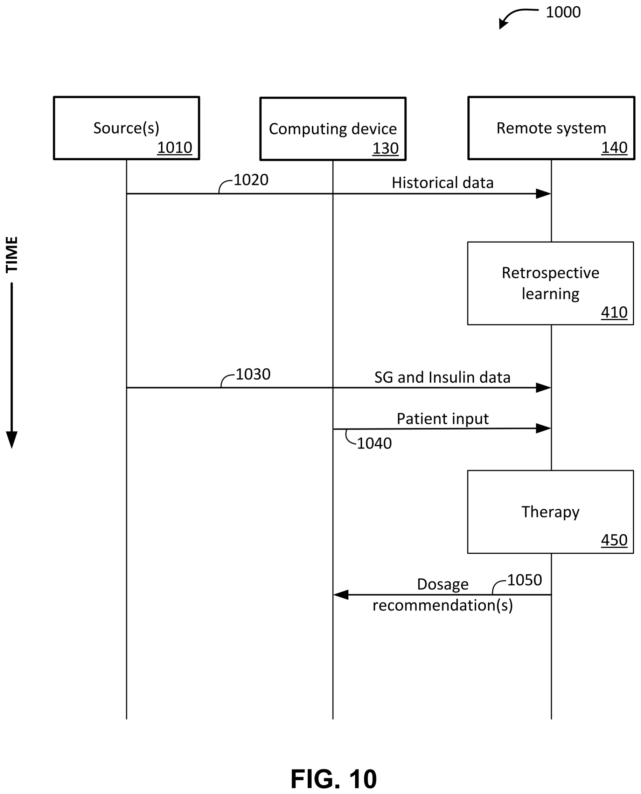

The output of the alerts may vary, depending on implementation. As noted above, the operations described in the embodiments herein may be performed by a local computing device (e.g., computing device 130 of FIG. 1, such as a smartphone or tablet), a remote system (e.g., remote or cloud computing system 140 of FIG. 1, such as one or more servers), or a combination thereof. For embodiments in which operations, including the generation of alerts, are performed by a local computing device (e.g., via one or more apps executed by the local device), the alerts may be communicated via the device's user interface, using audio alerts, visual/video alerts on the device's display, tactile (e.g., vibrating) feedback, etc. Depending on the desired functionality, a high alert recommendation may be associated with more urgent notifications (e.g., louder audio, stronger vibrations, more vibrant visual notifications, etc.). In contrast, a remedial alert recommendation may be associated with less urgent notifications (e.g., quieter, less urgent, and/or fewer notification forms). For embodiments in which the determination of the alert type is performed by a remote device, the alerts may be communicated to a local device in a message that includes an indication of the alert level (e.g., high alert versus remedial alert). FIG. 10, discussed below, provides an example of how communication may occur for implementations in which a remote system determines the dosage recommendations for a patient.

**FIG. 10** is a message flow diagram 1000, illustrating messages communicated between a local computing device 130, a remote system 140, and one or more sources 1010, according to some implementations. The message flow diagram 1000 illustrates an example embodiment of how the procedural flow 400 of FIG. 4 may be implemented when a remote system 140 performs the retrospective learning 410 and therapy 450 aspects of the procedural flow 400. (Of course, as noted elsewhere herein, embodiments are not so limited, and the computing device 130 may perform either/both of the retrospective learning 410 or therapy 450.) Messages between these devices are illustrated as arrows. Moreover, messaging may be performed via one or more communication protocols used in the communication links between the devices illustrated, and messages may be communicated via one or more intermediary devices, networks, etc. According to some embodiments, one or more of the illustrated messages may be sent in response to a request (not shown) by the receiving device. To avoid clutter, some communications and functions have been omitted or combined with other communications/functions (e.g., the communication of the administration of the bolus to the remote system 140, the communication specific to different types of dosage recommendations, etc.).

Of course, alternative embodiments may vary from the embodiment illustrated in terms of the number/content of messages, functions performed, timing, or the like. It can be noted that, in some applications, communication between the one or more sources 1010 and the remote system 140 may be relayed via the computing device 130. Further, in some applications, the computing device 130 may itself comprise a source (e.g., of user input from a patient, historical data obtained from an injection pen or other administration device, etc.). A person of ordinary skill in the art will appreciate such variations to the embodiments shown in FIG. 10.

The process in diagram 1000 may begin with the functionality shown by arrow 1020, in which the remote system 140 obtains historical data from one or more sources 1010. As noted in the embodiments described above, these sources may comprise one or more devices, sensors, logs, etc., from which retrospective learning 410 may be performed (e.g., in the manner described in the embodiments above). The remote system 140 then performs the retrospective learning 410 to determine a personalized physiological model for the patient, with which future dose recommendations can be determined.

As further illustrated, the remote system 140 then obtains SG and insulin data from one or more sources (e.g., a sensor device 120 and drug delivery device 110), as indicated by arrow 1030, as well as patient input 1040 (e.g., indicating or announcing the consumption of a meal). With this information, the remote system 140 can then perform the therapy 450 (e.g., in the manner described in the embodiments above). This can involve the determination of basal determination 460, meal bolus determination 470, and/or correction bolus determination 480, as previously discussed. These dosage recommendations may then be provided to the computing device 130, as indicated by arrow 1050. As noted above, dosage recommendations may be accompanied by a priority or alert indication. The computing device 130 may therefore provide a high alert or remedial alert (e.g., in the manner described above) depending on this indication.

As noted above, the processes described herein may be repeated and/or performed at different times, as needed. For example, retrospective learning 410 may be performed periodically (e.g., every day, every few days, every week, every few weeks, etc.) based on updated historical data (e.g., reflective of an updated look-back period or time window). In some embodiments, one or more aspects of therapy 450 may be performed based on updated SG and insulin data and/or patient input. For example, basal recommendations may be determined on a daily basis, and meal bolus recommendations may be determined on a per-meal basis (e.g., triggered by patient input announcing the consumption of a meal), and a correction bolus recommendation can be determined after a meal bolus is taken. As such, the devices may repeat some or all of the functionality associated with performing therapy 450 (including communicating the messages of arrows 1030-1050) more frequently than the functionality associated with performing retrospective learning 410.

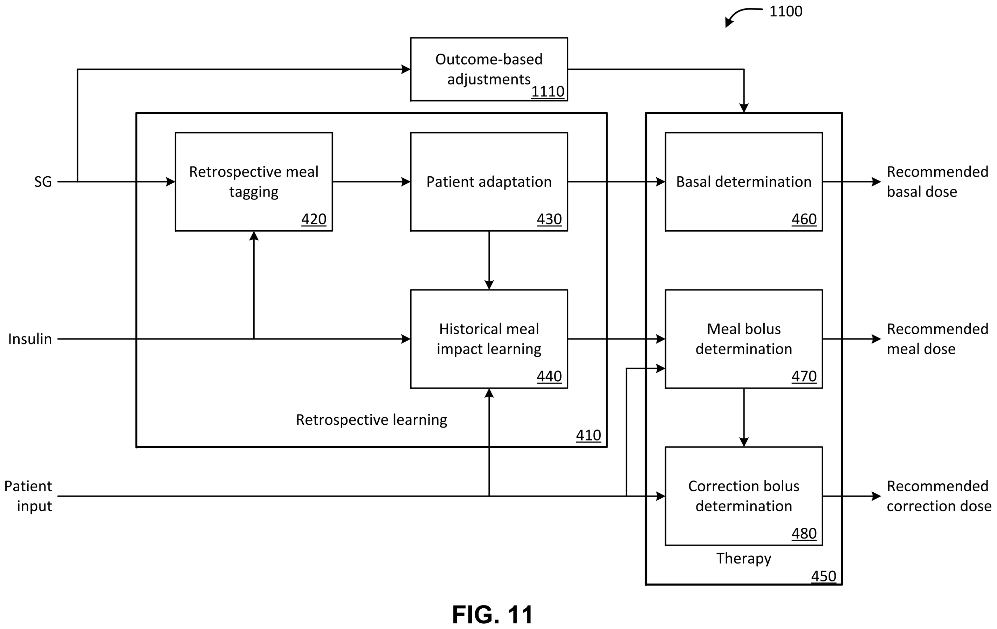

**FIG. 11** is a block diagram of a modified procedural flow 1100 showing a variation to the procedural flow 400 of FIG. 4 that may be implemented, according to one or more embodiments. In FIG. 11, various operations of the procedural flow 1100 correspond with counterpart operations in FIG. 4 and may be implemented in the same way as discussed above. In contrast to the procedural flow 400 of FIG. 4, however, the modified procedural flow 1100 includes the implementation of outcome-based adjustments 1110.

Outcome-based adjustments 1110 are a way in which one or more of the recommended doses may be further adjusted based on the outcome of previously-recommended doses. That is, despite the personalization that ongoing retrospective learning 410 provides, settings and parameters of the physiological model used to calculate recommended insulin doses may need to be further adjusted to help ensure titration of insulin dosages to ensure that glucose levels remain within a desirable range.

According to some embodiments, the outcome-based adjustments 1110 may be implemented by sectioning times of day (e.g., where different sections correspond to different meal times) for days in which previous dosage recommendations were provided. Each section may then be examined to determine whether glucose values remained within a desirable range (e.g., below 180 mg/dL and above 70 mg/dL, or based on user settings) for that section of time. If, for a given section, glucose values did not remain within the desirable range, future recommended dosages for that same section may be adjusted accordingly by altering pertinent settings and/or parameters used in dosage calculations. According to some embodiments, this process can be repeated on a daily basis to enable constant outcome-based adaptation.

Some embodiments may implement one or more additional features, depending on desired functionality. For example, in some embodiments, the outcome evaluation may be offset from the time where the adjustment is made. For instance, an evaluation on the data of a 10 am-2 pm window, may impact adjustments at 8 am-12 pm, therefore offset by +2 hours. Alternative embodiments may have different offsets (e.g., 1 hour, 1.5 hours, 2.5 hours, etc.). Similarly, according to some embodiments, the evaluation window may be longer or shorter than the adjustment window. Moreover, these evaluation offsets and/or evaluation window durations may vary for different times (sections) of the day. In some embodiments, the adjustment logic when low glucose (e.g. hypoglycemia) is observed in the evaluation window may be different from when high glucose (e.g. hyperglycemia) is observed. Additionally, this method may be applied to different insulin dose types separately, such as meal boluses, correction boluses, remedial boluses, and basal, which may result in different adjustments for different dose types. Additionally, or alternatively, variables for adjustment at each section of the day may include, but are not limited to, projection target, insulin sensitivity, meal parameters (including those from historical meal learning), carbohydrate ratios, and meal curve to carbohydrate ratio (e.g., the denominator average/median carb amount).

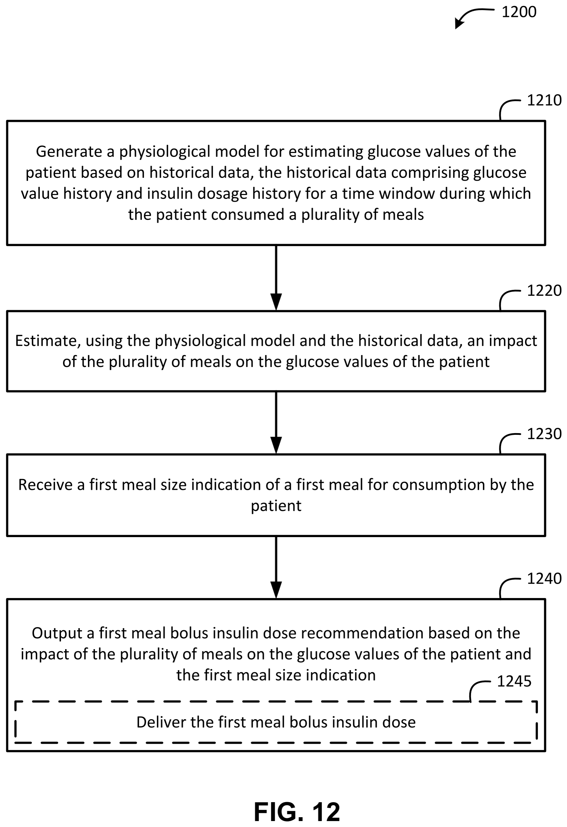

**FIG.** 12 is a flow diagram of a method 1200 of method of providing personalized insulin dosage recommendations for a patient, according to an embodiment. Means and/or structure for performing the functionality illustrated in one or more of the blocks shown in FIG. 12 may be performed by hardware and/or software components of a computer system (e.g., computing device 130, remote/cloud computing system140). Example components of a computer system are illustrated in FIG. 3, described above.

At block 1210, the functionality comprises generating a physiological model for estimating glucose values of the patient based on historical data, the historical data comprising glucose value history and insulin dosage history for a time window during which the patient consumed a plurality of meals. As noted in the embodiments above, the glucose value history may be indicative of glucose values of the patient measured during a time window during which the patient consumed a plurality of meals, and the insulin dosage history may be indicative of administration of insulin to the patient made during the time window. As noted in the embodiments above (e.g., with respect to block 420 of FIG. 4) embodiments may perform retrospective meal tagging of historical data. As such, some embodiments of the method 1200 may further comprise identifying a plurality of meal times based on the glucose value history and the insulin dosage history, where each meal time of the plurality of meal times corresponds to a time, during the time window, at which the patient consumed a respective meal of the plurality of meals. According to some aspects, the generation of the model may relate to functionality discussed above regarding blocks 420, 430, and 610 of the figures. In some embodiments, the method 1200 may further comprise determining an insulin sensitivity of the patient based on the glucose value history and insulin dosage history, where generating the physiological model is further based on the insulin sensitivity, fasting glucose, fasting insulin level, or any combination of these.

At block 1220, the functionality comprises estimating, using the physiological model and the historical data, an impact of the plurality of meals on the glucose values of the patient. According to some aspects, this may relate to functionality discussed above regarding blocks 440 and 610-650 of the figures. In some embodiments, estimating the impact of the plurality of meals on the glucose values of the patient may comprise generating a plurality of meal impact curves using the historical data. In such embodiments, each meal impact curve of the plurality of meal impact curves may be indicative of estimated glucose values of the patient for a respective meal of the plurality of meals, over a period of time following the respective meal. Examples of such functionality are discussed above (e.g., with regard to FIG. 7). Such embodiments may further comprise extracting, from the plurality of meal impact curves, a set of predetermined meal size indications representative of a plurality of different meal types, wherein the first meal size indication comprises a predetermined meal size indication from the set of predetermined meal size indications.

At block 1230, the functionality comprises receiving a first meal size indication of a first meal for consumption by the patient. As noted in the embodiments above, a meal size indication may comprise traditional size designations, meal type, carb count, etc. Further, according to some embodiments, meal size indications may be received via user/patient input or via some other source (e.g., an algorithm or machine-learning model). According to some embodiments, the first meal size indication is indicative of an estimated number of carbohydrates in the first meal.

At block 1240, the functionality comprises outputting a first meal bolus insulin dose recommendation based on the impact of the plurality of meals on the glucose values of the patient and the first meal size indication. As noted in the embodiments above (e.g., regarding FIG. 10) this may be done in different ways, which may depend on the type of device performing the functionality. As such, outputting the first meal bolus insulin dose recommendation may comprise at least one of: sending a message indicative of the first meal bolus insulin dose recommendation to a user device, or providing a message indicative of the first meal bolus insulin dose recommendation via a user interface. As shown by block 1245, according to some embodiments, outputting the first meal bolus insulin dose recommendation may include and/or be incorporated into the delivery of the bolus insulin dose (e.g., when the techniques are implemented within a pump or other automated insulin delivery system).

Alternative embodiments of the method 1200 may employ one or more of the following features, which correspond to features described in the embodiments above. For example, some embodiments of the method 1200 may further comprise, subsequent to outputting the first meal bolus insulin dose recommendation receiving an indication of a time of consumption of the first meal by the patient, obtaining post-meal data comprising one or more additional glucose values of the patient, measured subsequent to consumption of the first meal by the patient, calculating a correction bolus insulin dose based on the post-meal data, and outputting a recommendation comprising the correction bolus insulin dose based on a determination that the correction bolus insulin dose exceeds a predetermined threshold. Aspects of this functionality may correspond to features described above with respect to FIG. 9, for example. In such embodiments, outputting the recommendation may be further based on a determination that a threshold duration of time has passed since a meal bolus insulin dose was administered to the patient in conjunction with the consumption of the first meal by the patient, and the correction bolus insulin dose exceeds a higher-alert insulin threshold. Additionally, or alternatively, outputting the recommendation is further based on a determination that a threshold duration of time has passed since the time of the consumption of the first meal by the patient, and the correction bolus insulin dose does not exceed a higher-alert insulin threshold.

Alternative embodiments of the method 1200 also may one or more features related to the functionality described above with respect to the outcome-based adjustments of FIG. 11. For example, some embodiments may comprise, subsequent to outputting the first meal bolus insulin dose recommendation obtaining post-meal data comprising: a time of consumption of the first meal by the patient, one or more additional glucose values of the patient measured subsequent to consumption of the first meal by the patient, and an indication of a meal bolus insulin dose administered to the patient in conjunction with the consumption of the first meal by the patient. Embodiments may further comprise receiving a second meal size indication of a second meal for consumption by the patient, and outputting a second meal bolus insulin dose recommendation based on the post-meal data, the impact of the plurality of meals on the glucose values of the patient, and the second meal size indication. In such embodiments post-meal data may further comprises a remaining meal tracked based on model parameters used for determining the first meal bolus insulin dose recommendation.

The embodiments disclosed herein are examples of the disclosure and may be embodied in various forms. For instance, although certain embodiments herein are described as separate embodiments, each of the embodiments herein may be combined with one or more of the other embodiments herein. Specific structural and functional details disclosed herein are not to be interpreted as limiting, but as a basis for the claims and as a representative basis for teaching one skilled in the art to variously employ the present disclosure in virtually any appropriately detailed structure. Like reference numerals may refer to like elements throughout the description of the figures.

Any of the herein described techniques, operations, methods, programs, algorithms, or codes may be converted to, or expressed in, a programming language or computer program embodied on a computer, processor, or machine-readable medium. The terms "programming language" and "computer program," as used herein, each include any language used to specify instructions to a computer or processor, and include (but is not limited to) the following languages and their derivatives: Assembler, Basic, Batch files, BCPL, C, C+, C++, Delphi, Fortran, Java, JavaScript, machine code, operating system command languages, Pascal, Perl, PL1, Python, scripting languages, Visual Basic, metalanguages which themselves specify programs, and all first, second, third, fourth, fifth, or further generation computer languages. Also included are databases and other data schemas, and any other meta-languages. No distinction is made between languages that are interpreted, compiled, or that use both compiled and interpreted approaches. No distinction is made between compiled and source versions of a program. Thus, reference to a program, where the programming language could exist in more than one state (such as source, compiled, object, or linked) is a reference to any and all such states. Reference to a program may encompass the actual instructions and/or the intent of those instructions.

It should be understood that the foregoing description is only illustrative of the present disclosure. To the extent consistent, any or all of the aspects detailed herein may be used in conjunction with any or all of the other aspects detailed herein. Various alternatives and modifications can be devised by those skilled in the art without departing from the disclosure. Accordingly, the present disclosure is intended to embrace all such alternatives, modifications, and variances. The embodiments described with reference to the attached drawing figures are presented only to demonstrate certain examples of the disclosure. Other elements, steps, methods, and techniques that are insubstantially different from those described above and/or in the appended claims are also intended to be within the scope of the disclosure.

### Example Embodiments:

In view of this description, embodiments may include different combinations of features. Example embodiments are described in the following numbered clauses:
Clause 1: A method of providing personalized insulin dosage recommendations for a patient, the method comprising: generating a physiological model for estimating glucose values of the patient based on historical data, the historical data comprising glucose value history and insulin dosage history for a time window during which the patient consumed a plurality of meals; estimating, using the physiological model and the historical data, an impact of the plurality of meals on the glucose values of the patient; receiving a first meal size indication of a first meal for consumption by the patient; and outputting a first meal bolus insulin dose recommendation based on the impact of the plurality of meals on the glucose values of the patient and the first meal size indication.
Clause 2: The method of clause 1, further comprising identifying a plurality of meal times based on the glucose value history and the insulin dosage history, wherein each meal time of the plurality of meal times corresponds to a time, during the time window, at which the patient consumed a respective meal of the plurality of meals.
Clause 3: The method of either of clauses 1 or 2, further comprising determining an insulin sensitivity of the patient based on the glucose value history and insulin dosage history, wherein generating the physiological model is further based on the insulin sensitivity of the patient.
Clause 4: The method of any one of clauses 1-3, wherein estimating the impact of the plurality of meals on the glucose values of the patient comprises generating a plurality of meal impact curves using the historical data, wherein each meal impact curve of the plurality of meal impact curves is indicative of estimated glucose values of the patient for a respective meal of the plurality of meals, over a period of time following the respective meal.
Clause 5: The method of clause 4, further comprising extracting, from the plurality of meal impact curves, a set of predetermined meal size indications representative of a plurality of different meal types, wherein the first meal size indication comprises a predetermined meal size indication from the set of predetermined meal size indications.
Clause 6: The method of any one of clauses 1-5, wherein the first meal size indication is indicative of an estimated number of carbohydrates in the first meal.
Clause 7: The method of any one of clauses 1-6, further comprising, subsequent to outputting the first meal bolus insulin dose recommendation: receiving an indication of a time of consumption of the first meal by the patient; obtaining post-meal data comprising one or more additional glucose values of the patient, measured subsequent to consumption of the first meal by the patient; calculating a correction bolus insulin dose based on the post-meal data; and outputting a recommendation comprising the correction bolus insulin dose based on a determination that the correction bolus insulin dose exceeds a predetermined threshold.
Clause 8: The method of clause 7, wherein outputting the recommendation is further based on a determination that: a threshold duration of time has passed since a meal bolus insulin dose was administered to the patient in conjunction with the consumption of the first meal by the patient; and the correction bolus insulin dose exceeds a higher-alert insulin threshold.
Clause 9: The method of any one of clauses 7-8, wherein outputting the recommendation is further based on a determination that: a threshold duration of time has passed since the time of the consumption of the first meal by the patient, and the correction bolus insulin dose does not exceed a higher-alert insulin threshold.
Clause 10: The method of any one of clauses 1-9, further comprising, subsequent to outputting the first meal bolus insulin dose recommendation: obtaining post-meal data comprising: a time of consumption of the first meal by the patient, one or more additional glucose values of the patient measured subsequent to consumption of the first meal by the patient, and an indication of a meal bolus insulin dose administered to the patient in conjunction with the consumption of the first meal by the patient; receiving a second meal size indication of a second meal for consumption by the patient; and outputting a second meal bolus insulin dose recommendation based on the post-meal data, the impact of the plurality of meals on the glucose values of the patient, and the second meal size indication.
Clause 11: The method of clause 10, wherein the post-meal data further comprises a remaining meal tracked based on model parameters used for determining the first meal bolus insulin dose recommendation.
Clause 12: The method of any one of clauses 1-11, wherein outputting the first meal bolus insulin dose recommendation comprises at least one of: sending a message indicative of the first meal bolus insulin dose recommendation to a user device, or providing a message indicative of the first meal bolus insulin dose recommendation via a user interface, or automatically programming an insulin pump to deliver the dose recommendation.
Clause 13: The method of any one of clauses 1-12, wherein: the glucose value history is indicative of glucose values of the patient measured during a time window during which the patient consumed a plurality of meals; and the insulin dosage history is indicative of administration of insulin to the patient made during the time window.
Clause 14: A system, comprising: one or more processors; and one or more processor-readable media storing instructions which, when executed by the one or more processors, cause performance of: generating a physiological model for estimating glucose values of the patient based on historical data, the historical data comprising glucose value history and insulin dosage history for a time window during which the patient consumed a plurality of meals; estimating, using the physiological model and the historical data, an impact of the plurality of meals on the glucose values of the patient; receiving a first meal size indication of a first meal for consumption by the patient; and outputting a first meal bolus insulin dose recommendation based on the impact of the plurality of meals on the glucose values of the patient and the first meal size indication.
Clause 15: The method of clause 14, wherein the one or more processor-readable media further store instructions which, when executed by the one or more processors, cause performance of: identifying a plurality of meal times based on the glucose value history and the insulin dosage history, wherein each meal time of the plurality of meal times corresponds to a time, during the time window, at which the patient consumed a respective meal of the plurality of meals.
Clause 16: The method of either of clauses 14 or 15, wherein the one or more processor-readable media further store instructions which, when executed by the one or more processors, cause performance of: determining an insulin sensitivity of the patient based on the glucose value history and insulin dosage history, wherein generating the physiological model is further based on the insulin sensitivity of the patient.
Clause 17: The method of any one of clauses 14-16, wherein, to estimate the impact of the plurality of meals on the glucose values of the patient, the one or more processor-readable media further store instructions which, when executed by the one or more processors, cause performance of: generating a plurality of meal impact curves using the historical data, wherein each meal impact curve of the plurality of meal impact curves is indicative of estimated glucose values of the patient for a respective meal of the plurality of meals, over a period of time following the respective meal.
Clause 18: The method of clause 17, wherein the one or more processor-readable media further store instructions which, when executed by the one or more processors, cause performance of: extracting, from the plurality of meal impact curves, a set of predetermined meal size indications representative of a plurality of different meal types, wherein the first meal size indication comprises a predetermined meal size indication from the set of predetermined meal size indications.
Clause 19: The method of any one of clauses 14-18, wherein the first meal size indication is indicative of an estimated number of carbohydrates in the first meal.
Clause 20: The method of any one of clauses 14-19, wherein the one or more processor-readable media further store instructions which, when executed by the one or more processors, cause performance of, subsequent to outputting the first meal bolus insulin dose recommendation: receiving an indication of a time of consumption of the first meal by the patient; obtaining post-meal data comprising one or more additional glucose values of the patient, measured subsequent to consumption of the first meal by the patient; calculating a correction bolus insulin dose based on the post-meal data; and outputting a recommendation comprising the correction bolus insulin dose based on a determination that the correction bolus insulin dose exceeds a predetermined threshold.
Clause 21: The method of clause 20, wherein outputting the recommendation is further based on a determination that: a threshold duration of time has passed since a meal bolus insulin dose was administered to the patient in conjunction with the consumption of the first meal by the patient; and the correction bolus insulin dose exceeds a higher-alert insulin threshold.
Clause 22: The method of any one of clauses 20-21, wherein outputting the recommendation is further based on a determination that: a threshold duration of time has passed since the time of the consumption of the first meal by the patient, and the correction bolus insulin dose does not exceed a higher-alert insulin threshold.
Clause 23: The method of any one of clauses 14-22, wherein the one or more processor-readable media further store instructions which, when executed by the one or more processors, cause performance of, subsequent to outputting the first meal bolus insulin dose recommendation: obtaining post-meal data comprising: a time of consumption of the first meal by the patient, one or more additional glucose values of the patient measured subsequent to consumption of the first meal by the patient, and an indication of a meal bolus insulin dose administered to the patient in conjunction with the consumption of the first meal by the patient; receiving a second meal size indication of a second meal for consumption by the patient; and outputting a second meal bolus insulin dose recommendation based on the post-meal data, the impact of the plurality of meals on the glucose values of the patient, and the second meal size indication.
Clause 24: The method of any clause 23, wherein the post-meal data further comprises a remaining meal tracked based on model parameters used for determining the first meal bolus insulin dose recommendation.
Clause 25: The method of any one of clauses 14-24, wherein, to output the first meal bolus insulin dose recommendation, the one or more processor-readable media further store instructions which, when executed by the one or more processors, cause performance of at least one of: sending a message indicative of the first meal bolus insulin dose recommendation to a user device, or providing a message indicative of the first meal bolus insulin dose recommendation via a user interface.
Clause 26: The method of any one of clauses 14-25, wherein: the glucose value history is indicative of glucose values of the patient measured during a time window during which the patient consumed a plurality of meals; and the insulin dosage history is indicative of administration of insulin to the patient made during the time window.
Clause 27: An apparatus having means for performing the method of any one of clauses 1-13.
Clause 28: A non-transitory computer-readable medium storing instructions, the instructions comprising code for performing the method of any one of clauses 1-13.
Clause 29: A device comprising at least one memory and at least one processor communicatively coupled with the at least one memory, the at least one processor configured to perform, or cause the device to perform, the method of any one of clauses 1-13.

While several embodiments of the disclosure have been depicted in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

Further disclosed herein is the subject-matter of the following items:
1. A method of providing personalized insulin dosage recommendations for a patient, the method comprising:
   generating a physiological model for estimating glucose values of the patient based on historical data, the historical data comprising glucose value history and insulin dosage history for a time window during which the patient consumed a plurality of meals;
   estimating, using the physiological model and the historical data, an impact of the plurality of meals on the glucose values of the patient;
   receiving a first meal size indication of a first meal for consumption by the patient; and
   outputting a first meal bolus insulin dose recommendation based on the impact of the plurality of meals on the glucose values of the patient and the first meal size indication.
2. The method of item 1, further comprising identifying a plurality of meal times based on the glucose value history and the insulin dosage history, wherein each meal time of the plurality of meal times corresponds to a time, during the time window, at which the patient consumed a respective meal of the plurality of meals.
3. The method of item 1 or 2, further comprising determining an insulin sensitivity of the patient based on the glucose value history and insulin dosage history, wherein generating the physiological model is further based on the insulin sensitivity of the patient.
4. The method of item 1 or of any of items 1 to 3, wherein estimating the impact of the plurality of meals on the glucose values of the patient comprises generating a plurality of meal impact curves using the historical data, wherein each meal impact curve of the plurality of meal impact curves is indicative of estimated glucose values of the patient for a respective meal of the plurality of meals, over a period of time following the respective meal.
5. The method of item 1 or of any of items 1 to 4, wherein the first meal size indication is indicative of an estimated number of carbohydrates in the first meal.
6. The method of item 1 or of any of items 1 to 5, further comprising, subsequent to outputting the first meal bolus insulin dose recommendation:
   receiving an indication of a time of consumption of the first meal by the patient;
   obtaining post-meal data comprising one or more additional glucose values of the patient, measured subsequent to consumption of the first meal by the patient;
   calculating a correction bolus insulin dose based on the post-meal data; and
   outputting a recommendation comprising the correction bolus insulin dose based on a determination that the correction bolus insulin dose exceeds a predetermined threshold.
7. The method of item 1 or of any of items 1 to 6, further comprising, subsequent to outputting the first meal bolus insulin dose recommendation:
   obtaining post-meal data comprising:
      a time of consumption of the first meal by the patient,
      one or more additional glucose values of the patient measured subsequent to consumption of the first meal by the patient, and
      an indication of a meal bolus insulin dose administered to the patient in conjunction with the consumption of the first meal by the patient;
   receiving a second meal size indication of a second meal for consumption by the patient; and
   outputting a second meal bolus insulin dose recommendation based on the post-meal data, the impact of the plurality of meals on the glucose values of the patient, and the second meal size indication.
8. The method of item 1 or of any of items 1 to 7, wherein outputting the first meal bolus insulin dose recommendation comprises at least one of:
   sending a message indicative of the first meal bolus insulin dose recommendation to a user device, or
   providing a message indicative of the first meal bolus insulin dose recommendation via a user interface.
9. A system, comprising:
   one or more processors; and
   one or more processor-readable media storing instructions which, when executed by the one or more processors, cause performance of:
      generating a physiological model for estimating glucose values of a patient based on historical data, the historical data comprising glucose value history and insulin dosage history for a time window during which the patient consumed a plurality of meals;
      estimating, using the physiological model and the historical data, an impact of the plurality of meals on the glucose values of the patient;
      receiving a first meal size indication of a first meal for consumption by the patient; and
      outputting a first meal bolus insulin dose recommendation based on the impact of the plurality of meals on the glucose values of the patient and the first meal size indication.
10. The system of item 9, wherein the one or more processor-readable media further store instructions which, when executed by the one or more processors, cause performance of:
   identifying a plurality of meal times based on the glucose value history and the insulin dosage history, wherein each meal time of the plurality of meal times corresponds to a time, during the time window, at which the patient consumed a respective meal of the plurality of meals.
11. The system of item 9 or 10, wherein the one or more processor-readable media further store instructions which, when executed by the one or more processors, cause performance of:
   determining an insulin sensitivity of the patient based on the glucose value history and insulin dosage history, wherein generating the physiological model is further based on the insulin sensitivity of the patient.
12. The system of item 9 or of any of items 9 to 11, wherein, to estimate the impact of the plurality of meals on the glucose values of the patient, the one or more processor-readable media further store instructions which, when executed by the one or more processors, cause performance of:
   generating a plurality of meal impact curves using the historical data, wherein each meal impact curve of the plurality of meal impact curves is indicative of estimated glucose values of the patient for a respective meal of the plurality of meals, over a period of time following the respective meal.
13. The system of item 12, wherein the one or more processor-readable media further store instructions which, when executed by the one or more processors, cause performance of:
   extracting, from the plurality of meal impact curves, a set of predetermined meal size indications representative of a plurality of different meal types, wherein the first meal size indication comprises a predetermined meal size indication from the set of predetermined meal size indications.
14. The system of item 9 or of any of items 9 to 13, wherein the one or more processor-readable media further store instructions which, when executed by the one or more processors, cause performance of, subsequent to outputting the first meal bolus insulin dose recommendation:
   receiving an indication of a time of consumption of the first meal by the patient;
   obtaining post-meal data comprising one or more additional glucose values of the patient, measured subsequent to consumption of the first meal by the patient;
   calculating a correction bolus insulin dose based on the post-meal data; and
   outputting a recommendation comprising the correction bolus insulin dose based on a determination that the correction bolus insulin dose exceeds a predetermined threshold.
15. The system of item 14, wherein outputting the recommendation is further based on a determination that:
   a threshold duration of time has passed since a meal bolus insulin dose was administered to the patient in conjunction with the consumption of the first meal by the patient; and
   the correction bolus insulin dose exceeds a higher-alert insulin threshold.
16. The system of item 14 or 15, wherein outputting the recommendation is further based on a determination that:
   a threshold duration of time has passed since the time of the consumption of the first meal by the patient, and
   the correction bolus insulin dose does not exceed a higher-alert insulin threshold.
17. The system of item 9 or of any of items 9 to 16, wherein the one or more processor-readable media further store instructions which, when executed by the one or more processors, cause performance of, subsequent to outputting the first meal bolus insulin dose recommendation:
   obtaining post-meal data comprising:
      a time of consumption of the first meal by the patient,
      one or more additional glucose values of the patient measured subsequent to consumption of the first meal by the patient, and
      an indication of a meal bolus insulin dose administered to the patient in conjunction with the consumption of the first meal by the patient;
   receiving a second meal size indication of a second meal for consumption by the patient; and
   outputting a second meal bolus insulin dose recommendation based on the post-meal data, the impact of the plurality of meals on the glucose values of the patient, and the second meal size indication.
18. The system of item 17 or of any of items 9 to 17, wherein the post-meal data further comprises a remaining meal tracked based on model parameters used for determining the first meal bolus insulin dose recommendation.
19. The system of item 9 or of any of items 9 to 18, wherein, to output the first meal bolus insulin dose recommendation, the one or more processor-readable media further store instructions which, when executed by the one or more processors, cause performance of at least one of:
   sending a message indicative of the first meal bolus insulin dose recommendation to a user device, or
   providing a message indicative of the first meal bolus insulin dose recommendation via a user interface.
20. One or more non-transitory processor readable media storing instructions which, when executed by one or more processors, cause performance of:
   generating a physiological model for estimating glucose values of a patient based on historical data, the historical data comprising glucose value history and insulin dosage history for a time window during which the patient consumed a plurality of meals;
   estimating, using the physiological model and the historical data, an impact of the plurality of meals on the glucose values of the patient;
   receiving a first meal size indication of a first meal for consumption by the patient; and
   outputting a first meal bolus insulin dose recommendation based on the impact of the plurality of meals on the glucose values of the patient and the first meal size indication.

## Claims

1. A method of providing personalized insulin dosage recommendations for a patient, the method comprising:
generating a physiological model for estimating glucose values of the patient based on historical data, the historical data comprising glucose value history and insulin dosage history for a time window during which the patient consumed a plurality of meals;
estimating, using the physiological model and the historical data, an impact of the plurality of meals on the glucose values of the patient;
receiving a first meal size indication of a first meal for consumption by the patient; and
outputting a first meal bolus insulin dose recommendation based on the impact of the plurality of meals on the glucose values of the patient and the first meal size indication.

2. The method of claim 1, further comprising identifying a plurality of meal times based on the glucose value history and the insulin dosage history, wherein each meal time of the plurality of meal times corresponds to a time, during the time window, at which the patient consumed a respective meal of the plurality of meals,
and/or
further comprising determining an insulin sensitivity of the patient based on the glucose value history and insulin dosage history, wherein generating the physiological model is further based on the insulin sensitivity of the patient.

3. The method of claim 1 or 2, wherein estimating the impact of the plurality of meals on the glucose values of the patient comprises generating a plurality of meal impact curves using the historical data, wherein each meal impact curve of the plurality of meal impact curves is indicative of estimated glucose values of the patient for a respective meal of the plurality of meals, over a period of time following the respective meal.

4. The method of one of claims 1 to 3, wherein the first meal size indication is indicative of an estimated number of carbohydrates in the first meal.

5. The method of one of claims 1 to 4, further comprising, subsequent to outputting the first meal bolus insulin dose recommendation:
receiving an indication of a time of consumption of the first meal by the patient;
obtaining post-meal data comprising one or more additional glucose values of the patient, measured subsequent to consumption of the first meal by the patient;
calculating a correction bolus insulin dose based on the post-meal data; and
outputting a recommendation comprising the correction bolus insulin dose based on a determination that the correction bolus insulin dose exceeds a predetermined threshold.

6. The method of one of claims 1 to 5, further comprising, subsequent to outputting the first meal bolus insulin dose recommendation:
obtaining post-meal data comprising:
a time of consumption of the first meal by the patient,
one or more additional glucose values of the patient measured subsequent to consumption of the first meal by the patient, and
an indication of a meal bolus insulin dose administered to the patient in conjunction with the consumption of the first meal by the patient;
receiving a second meal size indication of a second meal for consumption by the patient; and
outputting a second meal bolus insulin dose recommendation based on the post-meal data, the impact of the plurality of meals on the glucose values of the patient, and the second meal size indication.

7. The method of one of claims 1 to 6, wherein outputting the first meal bolus insulin dose recommendation comprises at least one of:
sending a message indicative of the first meal bolus insulin dose recommendation to a user device, or
providing a message indicative of the first meal bolus insulin dose recommendation via a user interface.

8. A system, comprising:
one or more processors; and
one or more processor-readable media storing instructions which, when executed by the one or more processors, cause performance of:
generating a physiological model for estimating glucose values of a patient based on historical data, the historical data comprising glucose value history and insulin dosage history for a time window during which the patient consumed a plurality of meals;
estimating, using the physiological model and the historical data, an impact of the plurality of meals on the glucose values of the patient;
receiving a first meal size indication of a first meal for consumption by the patient; and
outputting a first meal bolus insulin dose recommendation based on the impact of the plurality of meals on the glucose values of the patient and the first meal size indication.

9. The system of claim 8, wherein the one or more processor-readable media further store instructions which, when executed by the one or more processors, cause performance of:
identifying a plurality of meal times based on the glucose value history and the insulin dosage history, wherein each meal time of the plurality of meal times corresponds to a time, during the time window, at which the patient consumed a respective meal of the plurality of meals,
and/or
wherein the one or more processor-readable media further store instructions which, when executed by the one or more processors, cause performance of:
determining an insulin sensitivity of the patient based on the glucose value history and insulin dosage history, wherein generating the physiological model is further based on the insulin sensitivity of the patient.

10. The system of claim 8 or 9, wherein, to estimate the impact of the plurality of meals on the glucose values of the patient, the one or more processor-readable media further store instructions which, when executed by the one or more processors, cause performance of:
generating a plurality of meal impact curves using the historical data, wherein each meal impact curve of the plurality of meal impact curves is indicative of estimated glucose values of the patient for a respective meal of the plurality of meals, over a period of time following the respective meal,
particularly wherein the one or more processor-readable media further store instructions which, when executed by the one or more processors, cause performance of:
extracting, from the plurality of meal impact curves, a set of predetermined meal size indications representative of a plurality of different meal types, wherein the first meal size indication comprises a predetermined meal size indication from the set of predetermined meal size indications.

11. The system of one of claims 8 to 10, wherein the one or more processor-readable media further store instructions which, when executed by the one or more processors, cause performance of, subsequent to outputting the first meal bolus insulin dose recommendation:
receiving an indication of a time of consumption of the first meal by the patient;
obtaining post-meal data comprising one or more additional glucose values of the patient, measured subsequent to consumption of the first meal by the patient;
calculating a correction bolus insulin dose based on the post-meal data; and
outputting a recommendation comprising the correction bolus insulin dose based on a determination that the correction bolus insulin dose exceeds a predetermined threshold.

12. The system of claim 11, wherein outputting the recommendation is further based on a determination that:
a threshold duration of time has passed since a meal bolus insulin dose was administered to the patient in conjunction with the consumption of the first meal by the patient; and
the correction bolus insulin dose exceeds a higher-alert insulin threshold, and/or wherein outputting the recommendation is further based on a determination that:
a threshold duration of time has passed since the time of the consumption of the first meal by the patient, and
the correction bolus insulin dose does not exceed a higher-alert insulin threshold.

13. The system of one of claims 8 to 12, wherein the one or more processor-readable media further store instructions which, when executed by the one or more processors, cause performance of, subsequent to outputting the first meal bolus insulin dose recommendation:
obtaining post-meal data comprising:
a time of consumption of the first meal by the patient,
one or more additional glucose values of the patient measured subsequent to consumption of the first meal by the patient, and
an indication of a meal bolus insulin dose administered to the patient in conjunction with the consumption of the first meal by the patient;
receiving a second meal size indication of a second meal for consumption by the patient; and
outputting a second meal bolus insulin dose recommendation based on the post-meal data, the impact of the plurality of meals on the glucose values of the patient, and the second meal size indication,
particularly wherein the post-meal data further comprises a remaining meal tracked based on model parameters used for determining the first meal bolus insulin dose recommendation.

14. The system of one of claims 8 to 13, wherein, to output the first meal bolus insulin dose recommendation, the one or more processor-readable media further store instructions which, when executed by the one or more processors, cause performance of at least one of:
sending a message indicative of the first meal bolus insulin dose recommendation to a user device, or
providing a message indicative of the first meal bolus insulin dose recommendation via a user interface.

15. One or more non-transitory processor readable media storing instructions which, when executed by one or more processors, cause performance of:
generating a physiological model for estimating glucose values of a patient based on historical data, the historical data comprising glucose value history and insulin dosage history for a time window during which the patient consumed a plurality of meals;
estimating, using the physiological model and the historical data, an impact of the plurality of meals on the glucose values of the patient;
receiving a first meal size indication of a first meal for consumption by the patient; and
outputting a first meal bolus insulin dose recommendation based on the impact of the plurality of meals on the glucose values of the patient and the first meal size indication.
